# EUROPEAN PATENT SPECIFICATION

(11) **EP 3 030 673 B1**
(45) Date of publication and mention of the grant of the patent: **04.10.2017**
(21) Application number: 14749795.2
(22) Date of filing: 06.08.2014
(51) Int. Cl.: C12Q 1/68

(54) **SCREENING AND IDENTIFICATION OF RESISTANT MARKERS IN CRUSTACEAN**
SCREENING UND IDENTIFIKATION VON RESISTENTEN MARKERN IN KRUSTENTIEREN
CRIBLAGE ET IDENTIFICATION DE MARQUEURS RÉSISTANTS DANS UN CRUSTACÉ

(30) Priority: 06.08.2013 NO 20131073
(43) Date of publication of application: 15.06.2016
(73) Proprietor: Patogen Analyse AS, 6025 Ålesund (NO); Bergen Teknologioverføring AS, 5008 Bergen (NO)
(72) Inventor: NILSEN, Frank, 5006 Bergen (NO); ESPEDAL, Per Gunnar, 5006 Bergen (NO)
(74) Representative: Onsagers AS
(86) International application number: PCT/EP2014/066898
(87) International publication number: WO 2015/018863

(56) References cited:
- EP-A1- 0 615 976
- VAN LEEUWEN THOMAS ET AL: "Acaricide resistance mechanisms in the two-spotted spider mite Tetranychus urticae and other important Acari: a review.", INSECT BIOCHEMISTRY AND MOLECULAR BIOLOGY AUG 2010, vol. 40, no. 8, August 2010 (2010-08), pages 563-572, XP002731810, ISSN: 1879-0240
- VAN LEEUWEN THOMAS ET AL: "Mitochondrial heteroplasmy and the evolution of insecticide resistance: non-Mendelian inheritance in action.", PROCEEDINGS OF THE NATIONAL ACADEMY OF SCIENCES OF THE UNITED STATES OF AMERICA 22 APR 2008, vol. 105, no. 16, 22 April 2008 (2008-04-22), pages 5980-5985, XP002731811, ISSN: 1091-6490
- TJENSVOLL KJERSTI ET AL: "Sequence variation in four mitochondrial genes of the salmon louse Lepeophtheirus salmonis.", DISEASES OF AQUATIC ORGANISMS 2 MAR 2006, vol. 68, no. 3, 2 March 2006 (2006-03-02), pages 251-259, XP002731812, ISSN: 0177-5103

### SEQUENCE LISTING

<110> PatoGen Analyse AS
<120> Screening and identification of resistant markers in crustaceans
<130> P23482PC00
<160> 20
<170> PatentIn version 3.5
<210> 1
   <211> 15445
   <212> DNA
   <213> Lepeophtheirus salmonis
<400> 1
   agccccctat ttaggtgcag ttaaataaat atatttattt aaattaacta tgtacctata 60
   tgagtcgccc tagaatcaat aaagttaagg caatgggagc atagtttaat tatagtgttg 120
   agaaaagggg aggattatac ctttatacgg acgtggccac gggcattcaa tggcaggttc 180
   tagacatata ttgaggaggg ggggggggag attaggtaat aaatttattt aaatttatta 240
   tatgctcgca aagttgaatt aggggtaata gggagtatac ggcttaacta tagtgtctag 300
   ggtaaatcag tattagaagc tacgtctccc aaagctataa gttctggaca actagcttct 360
   acccgaagaa ggtataaagt caagttcctt taataaagaa tattatgaaa ataatacaag 420
   ttcgaaagga tagaggacta agtaagattt aggcatttat aagcttagct aataccaggt 480
   ttaggttcta tttaataaaa gatttggaaa gtcaaggagg tgattactta tagagtaagg 540
   ttatcttctc cttcaaagta actttttccc tagggagtag ccaggtcagc ctttcaggct 600
   tataaaaaga ttagaataag cagggaaaat ctttgagtta aatttaaggt ttagaactaa 660
   agaggactat tatttttttt tttttttttt tgagcatgtc atgaaaagtc taggagagag 720
   gctgacgagc aatctatcct tctgagcagt tgtgtgaatt ctctgggctt agggagtaat 780
   taaatgcttt tatttaaatc aatctgcgaa gggggcaata aggtatgtta ttaataaata 840
   aggtttggag ctttaaatct aaagttgggg atatgtggac tgattaaccc agaataatat 900
   atatatatgc agtgctcaaa agcttgttgt caaaagaaag ccaggggcgg gcgcagagct 960
   gagacgcagt ccacgtgcta aaaaattact atgggggtag caaatatcaa taacaccgaa 1020
   gacaaaatat ggaaagctgc cgaaaagaaa aaagttaagg gaattgaagt taattgaata 1080
   agattttctc tagaatttcc ttgcgaagca actgaaaata attttatatt aaatagtatc 1140
   ccgagaatta aggcaatcga gattggaagt aggaaaagga ggaataaatt gagtaaagtt 1200
   atagtagaga gaatctcggc tataagattt atagtggggg gccctccagc attgagtaag 1260
   caagctaaga atcagatttt tactaacgta ggctgggtag taattatgcc cttatttaaa 1320
   ataatgtgac gagtcccaga ggacaagtaa aatataccag cggcgagaaa tatcaaagag 1380
   gaagtaaatc cgtgagccac aaagttaatt atggctctag aagttcctgt ggaagagaga 1440
   gagaaaactg tagcagaagc tatagacatg tgacctacgg aggagtaagc aataataacc 1500
   ttcatatcta cttgagttac gcaaagcatt atagccaagg ctgccacaac tacggagaaa 1560
   gagattatta ccttggtaaa aaatcctaaa aaaaaagagt ttatatggac aaatcgaata 1620
   agcccatacc cccccaactt aagcaaaact cctgccagga ttatagaccc cataagaggg 1680
   gcctctacgt gagctaaggg aagtcataaa tgtagggaaa agatgggcaa ctttacaaga 1740
   aatgctataa aagtcattaa aagaataata ggggaagggg aggatgattg ggcgataaaa 1800
   aaaaaagtga aataggagag atgggggcca gcaatgataa caaaaaggag gggcaaagag 1860
   cctattaaag tatatataat tattattttg ttagcaaaaa ttcgttctgg ctgatagcct 1920
   caacccaaaa tgattaaaaa aatagggata aggtttagct cgaaagcaat ataaaaaaaa 1980
   aggatattag agacagagaa agtcaaaact gaaaaaaaaa aaagtaagtt cgagtagaaa 2040
   gaggttaaag ccgctatttc gatagaaatc aacaaaagaa tagtcataga aagaataggg 2100
   gctctaacct cgtccaagaa aaaagaagag gagaggttaa aggagatgtc ataaatggga 2160
   atcagggtta taaaagaaaa aattaaagga aaagagataa gaaaattggg taatgaacta 2220
   ttaaaagctg taaaaaaagc tttaagtaaa aaggaggata taaggaaaaa aataagtttt 2280
   aaactagaag ttaattcata ataagttaat tttgagaaga ggtctagggg ctagggtgct 2340
   aattggactt acaagaagca gattttttat tttttggagg gctgtcgaac taaatactat 2400
   cctgtttatt gtgatgttgt ggttagaggg ggagaaaggc tccgagttga ggggaataaa 2460
   atacttctta attcaagggt ttaggtccct attaatagtc tgaggagtct tttattatat 2520
   aggattttta ataatagtag ggggtctttt aaagttagga gtgtttcctt tccatttatg 2580
   aagtctagat ttagcagtaa ggagaagatg ggattgctgg ctgtatttaa tgagtttaca 2640
   aaaagtccta cccctacacc tagtagtaaa tgtaagagaa ggaagtaggg ttttgagggg 2700
   tgtaactcta cttaatttgg tagttaggtg cttagggagg cttcataaaa gtatattaaa 2760
   ggaattatta ttttattctt cactatttaa cataagatta ttgttgctat tgaggagctc 2820
   gcactctatc ctagtttttt ttttccttat ttataggaga ttattaattc cttgttgggg 2880
   gattttaaaa aactccgagg tagactacct gcagcaagta catctaagaa gagggggatt 2940
   attagctttt ctgtcgctga gagggttccc tcctagtctg gggtttttgt acaagtatgg 3000
   gctattcaga cttttagtca gcttaaatta tggagtgtta tactgttgta gggttatact 3060
   cctcagtctc tttatatttt atatttattt aagagtgttt ttacagctga aattaatcgg 3120
   aagacgaaca tttatgccaa ggatgagggg gaaagggggc tatctacttg ccccccaagc 3180
   atttctaatg attaggggaa gattgattct atatagcttt taaaagacta ttttgaaaat 3240
   agtcaatgga gtttcttcaa agctatttgt ctaacgtaac tcgccaccct ttccatttag 3300
   ttgatgagtc tccttggcct ctattaaggt cttttgctgg agggtttgtg gctttaggcc 3360
   tattaggctt atttttaaac agaagaagga gcctaatttt tacttccctg atttttttag 3420
   ccctaattat aatccagtga tggttagatg tttcggttga gggcacttat caagggcttc 3480
   attcaaaagt cgttcaaatt gggctaagat gagggataat cttatttatt agatcagaag 3540
   ttttgttctt ctttagtttt ttttggggat attttcatag cagattagta ccaagagtag 3600
   aaattggagg aggggagtgg ccccctttag gggttacagg cctcagagct tgagaaatcc 3660
   ctttactcaa tacaatcatt ctgttgagat cgggggtgag agtcacttga agacatcaca 3720
   gattagtaat aggtaaacat caaaatgcat ctttaagatt actgattact gtattactag 3780
   gaggttattt tacttgtctg cagggagtag agtacttcga agcaagattc agtattagag 3840
   atagagtgta cggttcaact ttcttcttac taacaggatt ccatggttta cacgtgctag 3900
   taggggccac ttttttaggg gtctgttact tccgaatagt gtctgggcat tttggtaaca 3960
   gccaccattt tgggtttgaa gcggctgcgt ggtattgaca ctttgtagat gttgtttgac 4020
   tatttttatt tttgggggtt tactgcttag taaaaaaagg ctgttattag agtagtttat 4080
   caaaatatta actttgggag ttaaagaaaa ctttagttct ctgataggtt agtaatttat 4140
   acaaaatatt agcttgtcaa gccaaaaaaa aggaactcct tttagcctgg gaaacttaag      4200
   ttaataaaac tatcagtctt caaaactgac aataaaccct tttagcttcc gaattactat      4260
   aacttataaa gagtattaca ttgaagctgt aaaggaggct tagcctagta ataatttttt 4320
   tattcttatg ggtgttaggt ctaagcttta ctatttcctt attaatcata ttactaggtt 4380
   attctttaag acgaaggggg gaggcgaatt tagataagtt aagacctttt gagtgtggat 4440
   tcaatcctaa tgcaggtcat cgaaagatat tttctttaca tttcttccta gtctctttgg 4500
   tgtttttagt cttcgattta gagttggtaa ttcttttccc ctttctaagt gtaggaggag 4560
   tggggaggag ggtagaaaga tacttaaggt gtgttttatt tttgtttgta ttaggattag 4620
   gccttttata cgagtggtta ataaaaagtt tagagtgatg tgattaaatg taaaatacaa 4680
   atttttaatt taaatttccg ggggttatgg cttgttttga gaaggttttg agtactgggg 4740
   ggggttttaa tattcctttc agcttttagg agtttaaagg aatcctcaat actagtattg 4800
   agagcaaaac taagaatttc aaatttggaa ctagaagtca gagggttggg tgacttttac 4860
   tctatatcct tcaggggagt tgtgataata attaggggtt cagtgtatct ctactccctt 4920
   aggtatatag aaactgaaaa gtattttaat cgatttatga ctctcgttag gttgtttatt 4980
   tttagaatgc tagtgttaat ttacagaggg gatgtggtac aattaattct agggtgggat 5040
   ggcttaggtc ttaggtctta tttactagtt tgttactata ataataggaa ttcttctaat 5100
   gcaggggcac taacccttat actaaaccgt ttaggggatg tgggtttgtt tttaagaatt 5160
   tacgttatag taaattccag gcattcaatt aacaggttca taagcatctc tagggtaata 5220
   ggaaggctat tattatttgt agcttttact aaaagggctc aattgccctt tagggcttgg 5280
   ttacccgcag caatagctgc tcctactcct gtgtcttctc tagtccattc ctcaactttg 5340
   gttactgcag gagtctacgt attaattcga ttttatgaca ggctaatttc tgtgttatga 5400
   attaggttaa ttgtaggagt ctctactaga agattagcga ctttagtagc aatagctgaa 5460
   cgcgacataa aaaaaattgt agctttctca actttaagac atttaggaat tataataagt 5520
   attctgagat taggttgagt cgaactagcc tttaggcatt taattttcca tgcttttttt 5580
   aaagcgttat tatttctagt agtggggtat tgaatccaca gctcctgcgg ttatcaagac 5640
   ctattaaaaa ttaatttgtt gagtaggcaa gagccagtaa tcagaagtct tggcggagtt 5700
   tctataataa gtctttgtgg acttccctat ttaactgggt tttatagaaa agacttattt 5760
   atagaagcaa gtgtgcagtt taggagaagt cttcttggat taggttttat tttaagaagt 5820
   tgtgtaggtt cagtaatgta tagaattcgg atgtttctaa tagttaataa agtgggaagt 5880
   gtctcaaggc ttttaaattg aggagggtct tttaaatttt accagttaag tataagctta 5940
   ttatacttct tttccattat aggaggtaga ttttggagaa acttctgagt tacttccggg 6000
   agagttttat atgtcttttg agaagtaaaa atagtgatta ttaggttaat cacatgcagg 6060
   gggttgctag gagcttatct taggaataca ggggtaaaaa acaggttttt gagtaaacta 6120
   gggtacctaa gagacataag aatccggctc cccaacatcc tcagaaaatc cttgataagc 6180
   aaaagagtaa aattagataa ttcttttctt agcagccatt acaataaata ctacagagta 6240
   gaaggaggaa aatatttatt aaatagaggt aacttaaggt attctgaact aatcttagta 6300
   atattctttt tttatgtgat attatatgtg ctcagggggt aattgttcct caagcagaag 6360
   tagtatattg aaacataatt ttttgtctat gtgtcggtag tattggtgtt agggtattaa 6420
   aattatattt tttgtaaaga ggtaagctaa aaaagctgat agactcataa tctatctata 6480
   ggatcccttc tctttaaagc ttaaaagagg aaatttctaa tttctttatg gcttaggtcg 6540
   agcttttagc taataaaaag ggaattgcaa actccctatt cctcacaggg ttagcttgtt 6600
   taggtaattt atataaaatg cccaattgtg gagtgggtaa agcatcttgc cttaaacata 6660
   tgcctacttg atctcagttt agtttccaag atgcttcctc cccagtaata gaagagttta 6720
   ttttattcca tgatgtgatt atagtttgtt taggccttat cttagtatcc gtagggggta 6780
   tattaggggc agtgtttgta gaggggcctt atgtaggagg tttaatcgac ggagagtgac 6840
   tagagtgaat ttgaacagct ttacctggtt tagtactatt tggggtagct ataccctctt 6900
   tatctttatt atatatatac gatgaaataa caaattttga tttgggggta aaagtaaatg 6960
   ggtatcagtg atattggagc tacgagatac ccttagcaga gggccttgtg ttagattgtt 7020
   ttatggtccc cagaagggat acaaagttag gacaaatgcg cttacttgaa agagatgtgg 7080
   ctttaacttt acctattaaa agaatgctgc agttgatagt gtcttcaaag gatgtattac 7140
   atgcttgggc ggtccctagc ttaggggtta aaatagacgc tgtgccagga cgaattaata 7200
   ccctatcaat atatagattt cggccgggat tgtattttgg gcaatgttca gaaatttgtg 7260
   gggcacatca taggtttatg cctattttac tagagatagt aagggttgac gattttgcta 7320
   ggtgattaaa gttagtgaga gaagaataag cataaagtgc attgttatta aagtgttgta 7380
   ggtttttaat catcaggatt cctgtacttg taaatgttgc gtttattacc ctactagaac 7440
   gaaaaattat tggttactct caagcacgta aaggacccaa taaggtgtct tttttaggga 7500
   ttcttcagcc tttctccgat gctattaaat tatttgtaaa agaagtaggc aggttcagat 7560
   ttgtaaatta cagagtctac tggatttctc ccgtagtggg tctaagggtg gcactactaa 7620
   tctgaaatgt ctatccctta gagataaggt ttggatcttg aagaatttct tgacttggac 7680
   tgttagcggg gataagagta agagtttacc ccttatttct tagagggtga aggtctaatt 7740
   gtgtgtattc acatattgga ggagtgcgag gagttgctca agtaatctcc tacgaagtag 7800
   tttttactat cctagtattt tctttaataa gattaagagg aagcttaact ttagtgggga 7860
   gtatactatt tagggagtat ttttatgttt acatattaag tcctttatta gtgtacctat 7920
   gattcctaac aggtctagcg gagaccaacc gaactcccct tgatttctct gagggggaaa 7980
   gagagttagt gtccggattc aatacagagt atgggggcag ggggtttgct ttaattttta 8040
   tagcacagta tgcttcgatc ttattgttaa gaagtttatt tagattattt ttacttaaaa 8100
   gaagaggact tttgttttat gtaattagaa ggttaggagg gttcttgtga gtgtgaaggc 8160
   gggttactta ccctcggcac cggtatgact gattaataag attatgttga aaatctcttt 8220
   taccaagaat tctactcggg ggagggggaa taattttaat aataataaga atttaattgt 8280
   caactataaa aaagtgatta tgatcttgca accataaaga tattgggact ctatacctac 8340
   taagtggatt ttgatctgga ttagtgggtt tagctataag agtcatcatc cgtcttgagc 8400
   tgtctcagcc gggagcatat ttaggggatt cccaggttta taatgttatt gtaactgctc 8460
   atgccttcat tataatcttt ttcatagtaa tacctgtatt aattgggggt tttggaaatt 8520
   gattagttcc tttaatactg ggggcacctg acatagcttt cccccgctta aacaatataa 8580
   gattttggtt tttaataccc tctttgagtt tattgcttat aagggcatta gtagaaagtg 8640
   gtgcaggaac tgggtgaaca gtataccccc ccctgtcttc gggagttttt cactccgggg 8700
   cttcagtaga ttttgcgatt ttttccctcc acttagctgg agtgtcttct ttattagggg 8760
   ctgtaaactt tattagaact attacaaatt tacggtgctt gggactttta gtggggcaaa 8820
   tgccaatatt cccctgatcc gttttaatca ctgctgtgct tttactcttg tctttaccag 8880
   tgttagcggg agcaattact atacttctta ctgatcgaaa tttaaataca actttttttg 8940
   accctagagg tggaggggac cccattttat accagcattt attttgattt ttcgggcacc 9000
   ctgaagttta tattctaatt cttccagggt ttggattaat ctctcagatt attacccaag 9060
   aaacttgtaa agatgaggct tttggttcgc ttggtataat ttatgcaata gtagcaattg 9120
   gagtattggg atttattgtt tgagctcatc acatgtttac agttgggtta gactcagaca 9180
   ctcgagcgta ttttacggca gccactatag taattgctat ccctacaggg attaaagtat     9240
   ttagttgatt agggacttta tatggaagta agattattta taccccctca atttattggg     9300
   tggtagggtt tctgtttctt tttacagtgg gaggattaac aggtattgta ttagctaact     9360
   ctgccttgga tatgactttg catgacactt actatgtagt tgcccacttt cactatgttc     9420
   ttagaatagg agcagttttt gccctaatag cgggattcac tcactgatac ccccttttga     9480
   caggtttaag aatgaactta actataagga aagcgcagtt tatcctaata tttattggtg     9540
   taaatttaac gttttttcct atgtacttcc tagggttagc aggtatacct cggcggtata     9600
   gggattaccc agacttctat tatttgtgga atcaggtagc tagtttcggt tcttacctaa     9660
   ctttattttc tgtggtagta tttgtaggga taatttgaga gtctatagtt aggttacgac     9720
   cagtattagc ggtatttgat aggggaagat ccatcgagtt caaacatacg tgtcctccgt     9780
   ataaccattc ttataactcc tgcccccgac tgactttagc ataaataaat taatttaaaa     9840
   aaatacctaa aattagttat tttcctttcg tactaataag taagtaaaag ccctgagata     9900
   gaaaccaatc tgacttacgt cgatcttaac tcaaatcatg taagtacgaa aagtcgaaca     9960
   gacttaaaat aggaccttct gcagcactat attacttaat tcaacatcga ggtcacgaat    10020
   atcttttaaa taagagctct aaaagtatta cgcgctgtta tccctaaggt acctatgtgt    10080
   agcaaaataa ttggttcaga ataactaagg tttctttatt atattaagtg ggctatccca    10140
   gccaaacatg gtaaaggtct tagggtctta tcgtcttcgg agagtatatc agcgtttgta    10200
   ctgatagcta agattcaaga gataagaata aacatgatta accaatgcta gcttcataca    10260
   ggtttacaat taataaacta attattgcgc tactttaaag ccataaaaaa tcattcattg 10320
   ggcagataca tcagacacag tcttactatg tctttgttaa acagtttgga gaatctcaag 10380
   ttatttagtt tatatttaat ttaaaattaa taaaagttat ttaagtaagg aaataataaa 10440
   agtatattta ctagtgtaaa caagttataa ttctcagaat taactaccca aataataaga 10500
   gaagcttaat aaataaatta ggtcaataat aatttattat aacagagggg actattattc 10560
   ctgaaatatt tttagactag ttgctcacta atgtatactc agcataagct aaatacatta 10620
   ttttaaaatt cattctaaaa tgttttattt ctttgggaac cagattttaa aaagtaccat 10680
   atatcgtgtt catccttgtc ttatagctta gttttctacc taggcttaga gagaggatag 10740
   atatttttaa tcgggaaagt ttttattgat actaattgtt tacccatgat gcaaaaggta 10800
   agagttctta tactactttg aaggattact aataggggca gttcgcataa ctatcctatc 10860
   aagatggccc ctatacttcg ggcaccctat attttaaagg gggtggccca tgtctagcac 10920
   ttaaagctag tgcactttct ctgccacttt aaaaataatt tattaaataa aagattaagg 10980
   ataatctacg ataagcttct cccatagaga agtaaagcat aaagcagatt aagttactta 11040
   ttgagaataa tagcctatat aaaattctaa tttacatgca atgaaattgg actcgcagta 11100
   tatggattta ctaaataaaa aaagaagtag agatttttaa gttctgaata agaaagtgcc 11160
   agcattcgcg gttatacttt tagaaaaata ggcatttaaa tctcaaaaat taacatttca 11220
   tgaggtcaaa tttaaaaaat gttagtaaaa tttgatgaaa atagtccagt gaatcggatt 11280
   agtccccgag taataaagat aagtttttta gtagtactga atagaaaact aaactcaaat 11340
   ggcagaatta tctctcaaat tagggaaacc tgtattaaca gaatttactc tttagatttt 11400
   aattttttta aagtatggct gtcttacttt attcagaaat tgtcttagtc aagtaatatt 11460
   ttatataatt aatatttata ggttgcatta aattataaaa tagagtattt aatagtaagc 11520
   taaaaaacgc tgaatatttt agtaattagt tcaaattgtc cgtcactctc ttaggagata 11580
   agtcgtagca aagtaatagt tatggaaata gctattaaat ttcttttgaa gaaatttttt 11640
   ttaaattaat agttatttcg agagttgctt gtactataat tcttgggctg attcctagtc 11700
   ctttcatgtt gtgtttatta attgtagtat taactagagt tagatgtggg atagtaagga 11760
   ttttttgtag ccagtggtta agctacgctt tagtaattgt attcttgggg ggtataatag 11820
   tattgtttac ttacgcttca agaataagag tatcagataa attgacaaaa agaaggagat 11880
   gaaaaagact atttgtattt ctgattatgg ggttaacttc aagggccccc agaggattta 11940
   aagtttatat aggaagacta tattcaaata atgggggggt tatactttca atcttaacct 12000
   tttatttact cctgtgccta tttagagtgg taaaaatagt agaagtgaga aaaggggctt 12060
   tgatttctta gggttaacat aatttaattt gcatttaaag cttgccgttt ggctaacctt 12120
   acttttagag tgtaatcacg ttataatttt attataaaaa tagcttaaag cttaaaagta 12180
   tattttaagg tgtttagcac ataggatttt gagtcctaag gagttaagaa attatctaaa 12240
   ataattattt gggctaaagc atctagtatt tacaaaatac tcattttagt taaactacca 12300
   aataaagtta gcctaataag taaaaaaagt ggagttggat agttcagtag actttgaacg 12360
   gattgaagag ataagggcag caagccctat acatgcttca cagactataa ctgtcacaaa 12420
   taaaaatctg atagttgagt aggttatact gaagaccgaa aaaaaaatgc aaataggaat 12480
   tatggtagct gtaagagctt ctaataaaag tagcctgata agcaagtgcc ctttattaaa 12540
   taaaaatcac ctagaggtga ataaaggagg gagagctaac aagtataaac tgctaaggtg 12600
   aggtaaaaaa aaaatgagta caagagttgt taaaatagtt ctcatagagt ataaaaacaa 12660
   tagctgttag cttaaaagat aatctaataa agatgttaaa tttttacttt aattatggaa 12720
   ggtcctcttt taatccccta attatttaag gtcttgcatt agacgtttca ctgttaatga 12780
   aaagaagagt ttctctaaat attcagttag tataagaatt acacttaatt tccaattaag 12840
   agatttaaaa gttaaactga ctattaatca tagtaaattt tatatttcac ttacaatgaa 12900
   aaggtgccaa tggctgatta attcttatcg agctcctatc aattaagtag tttaataaaa 12960
   accttaaatt gtaaatttaa aattagattt tctcttaatt aaagccttag ggagtactaa 13020
   tgaattcttt attttccaga tttgatgcta acggattgac aaactgatac ttaccttctt 13080
   tagcaagagg tttattacta tttccaaggt attgattagc gggtaataca ttaggctcca 13140
   tgtgaagagg ggtttttagg gctattcatt ccgatgtaaa aattatattt agtgaagggg 13200
   cagggtttat gcttatgaga ttatttttaa gggttctaag aatgaatttt ctagggcttt 13260
   ttccgtacat ttttactagg agtaggcaca tctgtgtgag attaggtata gggctgcctt 13320
   tatgattagg cagtcaaata gtttgttgag tatacaggag agagcaaagg tttgcccacc 13380
   tagttccaga gggggctcct agaggtttaa ttcctctttt ggttttaatt gaaagaatta 13440
   gaaggttaat ccgacctcta accctaaggg ttcggcttat agctaatata attgcaggcc 13500
   acctactact aattttactt ggtagtaatg ctagagtggg gtcccctctt agaattctat 13560
   taactatgtt aggattatta ttcttagtcc tactagagtt aggggtgagt ctaattcaga 13620
   gatacgtttt tatattatta agctctttat atgttaggga gactcttcac taatgaattt 13680
   tgttactgtc ctagtttcga cctaggctta ggttttaacc agcaaaataa tctaattatt 13740
   tatgtttgtg taatgcgggc agttattacg cctgtcaaat gacttcaaac taataaaagt 13800
   ttagctcaca atccgtctga gtaaggtaag gacagtccta ctctgaagta taagacagta 13860
   aaaacttggc ccaggaaaat atagtttccc tctacaggtt ttgctccaat ccaggttaaa 13920
   agaaaaaaaa tcccgataaa agctcaaaac ataagcattt tcgaagggtt aaatgtagca 13980
   tgtacggcgg ctcttttatc ccccagcaaa aatggaaaaa aaaacaaaat tcttacagac 14040
   aaagctaaag ccactacccc ccccaactta tttgggatag accgcaaaat agcataagca 14100
   aacaaaaaat accattcagg ttgaatgtga gcaggagtta ctaaaggatt agctggaata 14160
   aagttttcag ggtctgctaa taatcaggga gagtaagaaa ctaaagctcc catgagacaa 14220
   aaaataagac cgaagccaaa ggcgtcttta aaggtatagt aagggtcaaa aggaatctta 14280
   taatagtttc ctggaacccc caatggattt gaggaccctg ttgaatgaag aaaaattgta 14340
   tgagctataa ctccaactgt ggacaaaaga ggaatgaggt aatgaagagc aaagaatcgc 14400
   cttaaagtag ctttgtccac cctaaacccc ccccaaagtc aaattacaat ttcgtcccca 14460
   ataccaggga tgacagaaaa tagatttgta ataaccgtag ctcctcagaa agatatctgg 14520
   ccccagggga gcacatagcc caaaaaagct ctggctatga ttaaaattaa aatcgtagag 14580
   cctacaactc aaacaggagt aagcttatac gaagaatagt aaaggccccg acccgtatga     14640
   atgtataaac acataaaaaa taacctcacg gtgttgctat gaatagctcg aattcctcac     14700
   ccataattaa catcccgcat gatatggtct acggataaaa acgctgtaga aatctctctt     14760
   gaataatgca tagccaaaaa aattccggtc ataatctggg taataagaca tagcccaagt     14820
   aaagacccaa agttccaccc cgacgaaata ttaactgggg tgggaagtgt tagaagagga     14880
   ttatagatag aaagagtttt ataagaattc ataaatggag ctttctggca gaaaaagtgc     14940
   gttaatttta gaaattaagt ataagagatt cttgtaagct acaacatact acttaatagt     15000
   atttattaaa ctcataggag ttaacaaata gtcttgatcc ataagattta ctgtgtgcaa     15060
   acggctagat tattagttta gtatattaac tagaacaaaa tagttagtta atatttatta     15120
   aaagtttaac caagttaagt ggaggaggct tctgtagtaa ccgcggagtt tggtttaatg     15180
   agaactctct taaagctaac cgaaactagg ttgatcttca aaatattttt taagattggt     15240
   ttaagctatg cacaagttgc taatatttat taaaagattt aaataagtag aaagtttaga     15300
   tttaatacta agaagaatta ctagagaata aagccagacc aaatatttat ttaaagattt     15360
   agataagtag ctacggctag gtggtgggga caaggtgcat aatcaataca agtgagttgg     15420
   gggggggggg tatggggggg gatta                                              15445
<210> 2
   <211> 1056
   <212> DNA
   <213> Lepeophtheirus salmonis
<400> 2
   ctgtctcagc cgggggcata tttaggggat tcccaggttt ataatgttat tgtaactgct 60
   catgccttca ttataatctt tttcatagta atacctgtat taattggggg gtttggaaat 120
   tgattagttc ctttaatact gggggcacct gacatagctt tccctcgctt aaacaatata 180
   agattttggt ttttaatacc ctctttgagt ttattggtta taagggcatt agtagaaagt 240
   ggtgcaggaa ctgggtgaac agtatacccc cccctgtctt cgggagtttt tcactccggg 300
   gcttcagtag attttgcgat tttttccctc cacttagctg gagtgtcttc tttattaggg 360
   gctgtaaact ttattagaac tattacaaat ttacggtgct tgggactttt agtggggcaa 420
   atgccaatat tcccctgatc cgttttaatc actgctgtgc ttttactctt gtctttacca 480
   gtgttagcgg gagcaattac tatacttctt actgatcgaa atttaaatac aacttttttt 540
   gaccctagag gtggagggga tcccatttta taccagcatt tattttgatt tttcgggcac 600
   cctgaagttt atattctaat tcttccaggg tttggattaa tctctcagat tattacccaa 660
   gaaacttgta aagatgaggc ttttggttcg cttggtataa tttatgcaat agtagcaatt 720
   ggagtattgg gatttattgt ttgagctcat cacatgttta cagttgggtt agactcagac 780
   actcgagcgt attttacggc agccactata gtaattgcta tccctacagg gattaaagta 840
   tttagttgat tagggccttt atatggaagt aagattattt ataccccctc aatttattgg 900
   gtggtagggt ttctgtttct ttttacagtg gggggattaa caggtattgt attagctaac 960
   tctgccttgg atatgacttt gcatgacact tactatgtag ttgcccactt tcactatgtt 1020
   cttagaatag gagcagtttt tcgacctagg cttagg 1056
<210> 3
   <211> 996
   <212> DNA
   <213> Lepeophtheirus salmonis
<400> 3
   ttttaaccag caaaataatc taattattta tgtttgtgta atgcgggcag ttattacgcc 60
   tgtcaaatga cttcaaacta ataaaagttt agctcacaat ccgtctgagt aaggtaagga 120
   cagccctact ctgaagtata agacagtaaa aacttggccc aggaaaatat agtttccctc 180
   tacaggtttt gctccaatcc aggttaaaag aaaaaaaatc ccgataaaag ctcaaaacat 240
   aagcattttc gaggggttaa atgtagcagg tacggcggtt cttttatccc ccagcaaaaa 300
   tggaaaaaaa aacaaaattc ttacagacaa agctaaagcc actacccccc ccaacttatt 360
   tgggatagac cgcaaaatag cataagcaaa caaaaaatac cattcaggtt gaatgtgagc 420
   aggagttact aaaggattag ctggaataaa gttttcaggg tctgctaata atcagggaga 480
   gtaagaaact aaagctccca tgagacaaaa aataagaccg aagccaaagg cgtctttaaa     540
   ggtatagtaa gggtcaaaag gaatcttata atagtttcct ggaaccccca atggatttga     600
   ggaccctgtt gaatgaagaa aaattgtatg agctataact ccaactgtgg acaaaagagg 660
   aatgaggtaa tgaagagcaa agaatcgcct taaagtagct ttgtccaccc taaacccccc 720
   ccaaagtcaa attacaattt cgtccccaat accagggatg acagaaaata gatttgtaat 780
   aaccgtagct ccccagaaag atatctggcc ccaggggagc acatagccca aaaaagctct 840
   ggctatgatt aaaattaaaa tcgtagagcc tacaactcaa acaggagtaa gcttatacga 900
   agaatagtaa aggccccgac ccgtatgaat gtataaacac ataaaaaata acctcgcggt 960
   gttgctatga atagctcgaa ttcctcaccc ataata 996
<210> 4
   <211> 1115
   <212> DNA
   <213> Lepeophtheirus salmonis
<400> 4
   ctaagtggat tttgatctgg attagtgggt ttagctataa gagtcatcat ccgtcttgag 60
   ctgtctcagc cgggagcata tttaggagat tcccaggttt ataatgttat tgtaactgct 120
   catgccttca ttataatctt tttcatagta atacctgtat taattggggg gtttggaaat 180
   tgattagttc ctttaatact gggggcacct gacatagctt tcccccgctt aaacaatata 240
   agattttggt ttttaatacc ctcttcgagt ttattactta taagggcatt agtagaaagt 300
   ggtgcaggaa ctgggtgaac agtatatccc cccctgtctt cgggagtttt tcactccggg 360
   gcttcagtag attttgcgat tttttccctc cacttagctg gagtgtcttc tttattaggg 420
   gctgtaaact ttattagaac tattacaaat ttacggtgct tgggactttt agtggggcaa 480
   atgccaatat tcccctgatc cgttttaatt actgctgtgc ttttactctt gtctttacca 540
   gtgttagcgg gagcaattac tatacttctt actgatcgaa atttaaatac aacttttttt 600
   gaccctagag gtggggggga tcctatttta taccagcatt tattttgatt tttcgggcac 660
   cctgaagttt atattctaat tcttccaggg tttgggttaa tctctcagat tattacccaa 720
   gaaacttgta aagatgaggc ttttggttcg cttggtataa tttatgcaat agtagcaatt 780
   ggagtattgg ggtttattgt ttgagctcat cacatgttta cagttgggtt agactcagac 840
   actcgagcgt attttacggc agccactata gtaattgcta tccctacagg gattaaagta 900
   tttagttgat tagggacttt atatggaagt aagattattt ataccccctc aatttattgg 960
   gtggtagggt ttctgtttct ttttacagtg ggaggattaa caggtattgt attagctaac 1020
   tctgccttgg atatgacttt gcatgacact tactacgtag ttgcccactt tcactatgtt 1080
   cttagaatag gggcagtttt tgccctaata gcggg 1115
<210> 5
   <211> 1255
   <212> DNA
   <213> Lepeophtheirus salmonis
<400> 5
   caaaataatc taattattta tgtttgtgta atgcgggcag ttattacgcc tgtcaaatga 60
   cttcaaacta ataaaagttt agctcacaat ccgtctgagt aaggtaagga cagccctact 120
   ctgaagtata agacagtaaa aacttggccc aggaaaatat agtttccctc tacaggtttt 180
   gctccaatcc aggttaaaag aaaaaaaatc ccgataaaag ctcaaaacat aagtattttc 240
   gaggggttaa atgtagcagg tacggcggtt cttttatccc ccagcaaaaa tgggaaaaaa 300
   aacaaaattc ttacagacaa agctaaagcc actacccccc ctaacttatt tgggatagac 360
   cgcaaaatag cataagcaaa caaaaaatac cattcaggtt gaatgtgagc aggagttact 420
   aaaggattag ctggaataaa gttttcaggg tctgctaata atcagggaga gtaagaaact 480
   aaagctccca tgagacaaaa aataagaccg aagccaaagg cgtctttaaa ggtatagtaa        540
   gggtcaaaag gaatcttata atagtttcct ggaaccccca atggatttga ggaccctgtt        600
   gaatgaagaa aaattgtatg agctataact ccaactgtgg acaaaagagg aatgaggtaa 660
   tgaagagcaa agaatcgcct taaagtagct ttgtccaccc taaaaccccc tcaaagtcaa 720
   attacaattt cgtccccaat accagggatg acagaaaata gatttgtaat aaccgtagct 780
   cctcagaaag atatctggcc ccaggggagc acatagccca aaaaagctct ggctatgatt 840
   aaaattaaaa tcgtagagcc tacaactcaa acaggagtaa gcttatacga agaatagtaa 900
   aggccccgac ccgtatgaat gtataaacac ataaaaaata acctcgcggt gttgctatga 960
   atagctcgaa ttcctcaccc ataattaaca tcccgcatga tatggtctac ggataaaaac 1020
   gctgtagaaa tttctcttga ataatgcata gccaaaaaaa ttccggtcat aatttgggta 1080
   ataagacata gcccaagtaa agacccaaag ttccaccccg acgaaatatt aactggggtg 1140
   ggaagtgtta gaagaggatt atagatagaa agagttttat aagaattcat aaatggagct 1200
   ttctggcaga aaaagtgcgt taattttaga aattaagtat aagagattct tgtaa 1255
<210> 6
   <211> 18
   <212> DNA
   <213> Lepeophtheirus salmonis
<400> 6
   ttaaacaata taagattt 18
<210> 7
   <211> 24
   <212> DNA
   <213> Lepeophtheirus salmonis
<400> 7
   tagaaagtgg tgcaggaact gggt 24
<210> 8
   <211> 26
   <212> DNA
   <213> Lepeophtheirus salmonis
<400> 8
   gggcaccctg aagtttatat tctaat 26
<210> 9
   <211> 26
   <212> DNA
   <213> Lepeophtheirus salmonis
<400> 9
   aaccaaaagc ctcatcttta caagtt 26
<210> 10
   <211> 21
   <212> DNA
   <213> Lepeophtheirus salmonis
<400> 10
   aggttaaaga aaaaaaatcc c 21
<210> 11
   <211> 21
   <212> DNA
   <213> Lepeophtheirus salmonis
<400> 11
   taaatgtagc agtacggcgg t 21
<210> 12
   <211> 19
   <212> DNA
   <213> Lepeophtheirus salmonis
<400> 12
   gcaggtacgg cggttcttt 19
<210> 13
   <211> 28
   <212> DNA
   <213> Lepeophtheirus salmonis
<400> 13
   gctttagctt tgtctgtaag aattttgt 28
<210> 14
   <211> 27
   <212> DNA
   <213> Lepeophtheirus salmonis
<400> 14
   ttcttacaga caaagctaaa gccacta 27
<210> 15
   <211> 25
   <212> DNA
   <213> Lepeophtheirus salmonis
<400> 15
   agtaactcct gctcacattc aacct 25
<210> 16
   <211> 16
   <212> DNA
   <213> Lepeophtheirus salmonis
<400> 16
   cctcttygag tttatt 16
<210> 17
   <211> 17
   <212> DNA
   <213> Lepeophtheirus salmonis
<400> 17
   ttccagggtt tggrtta 17
<210> 18
   <211> 19
   <212> DNA
   <213> Lepeophtheirus salmonis
<400> 18
   caaaacataa gycattttc 19
<210> 19
   <211> 15
   <212> DNA
   <213> Lepeophtheirus salmonis
<400> 19
   cagcaaaaat ggraa 15
<210> 20
   <211> 14
   <212> DNA
   <213> Lepeophtheirus salmonis
<400> 20
   ccccccyaac ttat 14

## Description

### Field of the invention

The present disclosure relates to a method for the detection of resistance of pests to pyrethroid chemotherapeutics, in particular to a method of determining single nucleotide polymorphisms associated with pyrethroid resistance in arthropods, in particular crustaceans, in particular copepods, and the use of such SNP's in a screening method for determining whether such pests are sensitive to, or resistant to, pyrethroids.

### Background of the invention

Currently, pyrethroids are the most used insecticides against arthropod plagues in agriculture and livestock as well as in the control of vectors of veterinary and human health importance.

Examples of such pests are species of crustacean, and in particular in the class copepod known as sea lice. Infestations of the parasitic copepods *Lepeophtheirus salmonis* and *Caligus spp.,* commonly referred to as sea lice, represent a major challenge to commercial salmon aquaculture. Control measures have been reliant upon the use of a limited number of chemotherapeutants, in particular pyrethroid compounds, since the 1970's, and reduced efficacy has now been reported for the majority of these compounds. Reduced sensitivity and potential resistance to currently available medicines are constant threats to maintaining control of sea lice populations in Atlantic salmon farms. Today, resistance against most of the available treatment options is widespread in almost every region where salmonids are cultured in sea water. Double- or triple resistance, in which sea lice is resistant to more than one type of chemotherapeutant, have been verified.

Drug resistance in sea lice has been detected by various types of bioassays as a significant increase in EC50/LC50. Although the bioassays can detect any type of resistance to a given drug, such methods are not very accurate or sensitive.

The genomes of all organisms undergo spontaneous mutations during their continuing evolution, forming variant forms of progenitor genetic sequences. A mutation may results in an evolutionary advantage or disadvantage relative to a progenitor form or may be neutral. A variant that result in an evolutionary advantage may eventually be incorporated in many members of the species and may thus effectively become the progenitor form. Furthermore, often various variant forms survive and coexist in a species population. The coexistence of multiple forms of a genetic sequence gives rise to genetic polymorphism, including single-nucleotide polymorphisms (SNPs).

A single-nucleotide polymorphism (SNP) is a DNA sequence variation occurring when a single nucleotide - A, T, C or G - in the genome (or other shared sequence) differs between members of a biological species or paired chromosomes in an organism. For example, two DNA fragments from different individuals, AAGCCTA to AAGCTTA, contain a difference in a single nucleotide, commonly referred to as two alleles. Almost all common SNPs have only two alleles. The genomic distribution of SNPs is not homogenous; SNPs usually occur in non-coding regions more frequently than in coding regions or, in general, where natural selection is acting and fixating the allele of the SNP that constitutes the most favorable genetic adaptation (Adapted from Wikipedia).

Of particular relevance is the fact that occasionally a SNP may occur in a pest organism that provides that organism with resistance to known pesticides.

Up to date, however, no SNPs specifically associated with insecticide resistance in sea lice have been reported. In fact, no such SNPs have been identified for any crustacean species (Arthropode Pesticide Resistance Database, http://www.pesticideresistance.com/).

It is known in the art that in some species of arthropods, specific mutations in the gene coding for the voltage gated sodium channel have been reported to result in an altered function of this channel, resulting in a decreased ability for pyrethroids to interfere with its function (Zlotkin E, (1999). The insect voltage-gated sodium channel as target of insecticides. Annu Rev Entomol. 44:429-55).

Previously, non-mendelian inheritance of resistance by spider mites towards a hydrazine carbazate derivate have been linked to mutations in mitochondrial DNA of the spider mite (Van Leeuwen, T., Vanholme, B., Van Pottelberge, S., Van Nieuwenhuyse, P., Nauen, R., Tirry, L., & Denholm, I. (2008). Mitochondrial heteroplasmy and the evolution of insecticide resistance: non-Mendelian inheritance in action. Proceedings of the National Academy of Sciences of the United States of America, 105(16), 5980-5). Several factors are drastically different however between the observations regarding spider-mites, and inherited resistance towards pyrethroids in crustaceans, in particular copepods such as seal lice. First of all, the spider-mite and sea lice are distantly related. Also, as the present inventors have observed, SNPs in non-coding regions of the mitochondrion is important for the regulation of resistance towards pyrethroids, while in the spider-mite it was highly conserved coding-regions that were observed to be of importance. No previous study has ever described non-coding regions of the mitochondrial DNA to be of importance to pesticide resistance in any species.

Genes encoded by mitochondrial DNA (mtDNA) exist in large numbers per cell but can be selected very rapidly as a result of unequal partitioning of mtDNA between germ cells during embryogenesis. Each mitochondrion is estimated to contain 2-10 mtDNA copies (Wiesner RJ, Ruegg JC, Morano I (1992). "Counting target molecules by exponential polymerase chain reaction, copy number of mitochondrial DNA in rat tissues". Biochim Biophys Acta. 183 (2): 553-559. doi:10.1016/0006-291X(92)90517-O. PMID 1550563.), and each cell contains several mitochondria. However, empirical studies of this "bottlenecking" effect are rare because of the apparent scarcity of heteroplasmic individuals possessing more than one mtDNA haplotype. Previously, Van Leeuwen et al, (2008)) (Van Leeuwen, T., Vanholme, B., Van Pottelberge, S., Van Nieuwenhuyse, P., Nauen, R., Tirry, L., & Denholm, I. (2008). Mitochondrial heteroplasmy and the evolution of insecticide resistance: non-Mendelian inheritance in action. Proceedings of the National Academy of Sciences of the United States of America, 105(16), 5980-5) have reported an example of insecticide resistance in an arthropod pest (*Tetranychus urticae*) being controlled by coding-regions of the mtDNA and on its inheritance in a heteroplasmic mite strain. The present invention is believed to represent the first example of insecticide resistance in an arthropod pest, in particular in a crustacean pest, and more particularly in a copepod pest such as sea lice (*L*. *salmonis*) being controlled by non-coding regions of the mtDNA and on its inheritance in a heteroplasmic lice strain. Also, this is the first report of resistance towards pyrethroids being controlled by mitochondrial genes in an arthropod.

In spider mite, resistance to the insecticide bifenazate is highly correlated with remarkable mutations in cytochrome b, a mitochondrially encoded protein in the respiratory pathway (Van Leeuwen, T., Vanholme, B., Van Pottelberge, S., Van Nieuwenhuyse, P., Nauen, R., Tirry, L., & Denholm, I. (2008). Mitochondrial heteroplasmy and the evolution of insecticide resistance: non-Mendelian inheritance in action. Proceedings of the National Academy of Sciences of the United States of America, 105(16), 5980-5). Four sites in the Qₒ site that are absolutely conserved across fungi, protozoa, plants, and animals are mutated in resistant mite strains. The same conserved sites are also present in the L. salmonis mtDNA, but these sites are not mutated in any strains of the L. salmonis mtDNA, and the resistance traits are not linked to these sites as they are in the spider mite.

Partially resistant strains, consisting of heteroplasmic individuals, transmit their resistant and susceptible haplotypes to progeny in highly variable ratios consistent with a sampling bottleneck. Insecticide selection on heteroplasmic individuals favors those carrying resistant haplotypes at a frequency of 60% or more. This combination of factors enables very rapid evolution and accounts for mutations being fixed in most field-collected resistant strains (Van Leeuwen, T., Vanholme, B., Van Pottelberge, S., Van Nieuwenhuyse, P., Nauen, R., Tirry, L., & Denholm, I. (2008). Mitochondrial heteroplasmy and the evolution of insecticide resistance: non-Mendelian inheritance in action. Proceedings of the National Academy of Sciences of the United States of America, 105(16), 5980-5).

Of particular note, reciprocal crosses between susceptible and resistant se lice showed resistance to be inherited only maternally; offspring of resistant females were strongly resistant, but those of susceptible females remained fully susceptible. Such complete uniparental inheritance is unprecedented for insecticide resistance and suggested mitochondrial control as one possible explanation. The fact that non-coding regions of the mtDNA controls resistance is highly surprising.

Mitochondria contain few genes as compared to cell nuclei, but they exhibit some intriguing evolutionary characteristics. Their inheritance is essentially non-Mendelian in that recombination through meiosis is lacking and transmission to offspring is predominantly uniparental. In contrast to nuclear DNA, in which there are only two copies of each gene per cell, the mitochondrial DNA (mtDNA) copy number in somatic cells is generally in the range of 10³-10⁴ copies per cell (Legros F , Malka F , Frachon P , Lombes A , Roja M (2004) Organization and dynamics of human mitochondrial DNA. J Cell Sci 117:2653-2662). Higher mutation rates in animal mtDNA together with limited DNA repair mechanisms render these genes susceptible to rapid evolution through random drift or natural selection. As a consequence, mtDNA is highly polymorphic and is widely used for studies in systematics and population genetics. However, most genetic variation within a species exists between individuals, and the occurrence of more than one mtDNA sequence variant in a single individual (heteroplasmy) is relatively rare (Jenuth JP , Peterson AC , Fu K , Shoubridge EA (1996) Random genetic drift in the female germ line explains the rapid segregation of mammalian mitochondrial DNA. Nat Genet 14:146-151.). No previous study has uncovered resistance traits connected to mutations in non-coding regions of the mitochondrion, but we strongly believe that this, as in the L. salmonis, is an important mechanism for the development of resistance in arthropods.

Another mechanism which has been suspected to contribute in the resistance towards pyrethroids is enhanced detoxification capability by the parasite. From other arthropods, increased activities of monooxydases and unspecific esterases have been identified as mechanisms in question. However, esterases seem to play an insignificant role in sea lice, as the parasite has a very low background activity of these enzymes. On the other hand, unspecific monooxidases seem to play a significant role, indicated by a correlation between enzyme activity and reduced sensitivity.

The mitochondrial genes COI, A6, Cytb and 16sRNA of *Lepeophtheirus salmonis* has shown to display high levels of polymorphisms (Tjensvoll et al., 2006, Diseases of Aquatic Organisms, vol, 68, pp. 251 - 259), resulting in a significant number of haplotypes. However, the prior art is silent as to whether the large number of polymorphic sites have had any effects on the characteristics of the sea lice.

Pyrethroids are one type of chemotherapeutics used for delousing of commercial salmon aquaculture sites infested with sea lice. In Norway, the synthetic deltamethrin (AlphaMax^{™}) and cis-cypermethrin (BetaMax^{™}) are the most widely used pyrethroids. In Scotland and Ireland, cypermethrin (ExisTM) has been commonly used. The pyrethroids affect the sodium channels thus inhibiting the transmittal of nerve impulses in the synapses. Failure of pyrethroid treatment has been reported in Norway (deltamethrin and cis-cypermethrin) since turn of the century (Sevatdal and Horsberg, 2000, Norsk Fiskeoppdrett, vol. 12, pp. 34-35), and reduced sensitivity towards pyrethroids was documented in 2003 (Sevatdal og Horsberg, 2003, Aquaqulture, vol. 218, pp. 21-31).

Efficient and sensitive methods for diagnosing resistance are crucial in order to manage and control drug resistance. Early detection of reduced sensitivity to a chemical can enable effective countermeasures to be enforced at a time point when these have a greater probability of being effective. Therefore, accurate and speedy identification of pyrethroid resistant pests is crucial. Detection of resistance prior to treatment, and the use of such analyses after treatment to evaluate treatment efficacy constitutes an important determinant for an integrated pest management (IPM) system.

### Summary of invention

The present invention is based on the surprising finding that resistance towards pyrethroid chemotherapeutics commonly used to combat arthropod pests, in particular the copepod pest sea lice, is linked to mutations in the mitochondrion genome. More particularly, the present invention is based on the identification of novel single-nucleotide polymorphisms (SNPs) in the mitochondrion genome of the sea lice (*Lepeophtheirus salmonis*) shown to be involved in the resistance towards pyrethroid-based chemotherapy. Specifically, the present inventors have identified two pyrethroid resistance-associated SNPs located in the mitochondrial Col gene (T8605C, A9035G) and three pyrethroid resistance-associated SNP in the mitochondrial Cyp b gene (C13957T, A14017G and C14065T).

It is further noted that the identification of the SNPs involved in pyrethroid resistance in the mitochondrial genome proved to be challenging due to unexpected characteristics such as numerous repeats etc. of the non coding parts of the mitochondrial genom (D-loop).

The present invention thus provides an in vitro method for determination of pyrethroid resistance in arthropod pests, in particular crustaceans, in particular copepods, comprising the steps of detecting one or more single nucleotide polymorphism (SNP) associated with pyrethroid resistance in the mitochondrial genome of the organism to be analyzed, as defined in the claims. According to one aspect, the pest is a member of a species having ZZ-ZO-type sex determination.

According to another aspect of the invention, the single nucleotide polymorphism (SNP) associated with pyrethroid resistance is present in the coding region of a gene coding for Cytochrom B.

According to another aspect of the invention, the single nucleotide polymorphism (SNP) associated with pyrethroid resistance is present in the coding region of a gene coding for Col.

According to another aspect of the invention, single nucleotide polymorphism (SNP) associated with pyrethroid resistance is present in a regulatory non-coding part of the mitochondria.

According to another aspect, the invention provides for isolated DNA sequences of mitochondrial DNA comprising a single nucleotide polymorphism (SNP) associated with pyrethroid resistance.

According to another aspect, the invention provides a method of identifying single nucleotide polymorphisms (SNP) associated with pyrethroid resistance in an arthropod pest, in particular a crustacean, in particular a copepod, in particular sea lice comprising the steps of
a) collecting a sample of the organism from its natural environment;
b) isolating mitochondrial genomic material from the collected organism
c) identifying the nucleotide at the polymorphic site of the isolated genomic material,
d) comparing the nucleotide polymorphic site of the isolated mitochondrial genomic material with a standard mitochondrial nucleic acid sequence from a member of the species known to be susceptible to pyrethroids, wherein the collected organism is determined to be resistant to pyrethroids if the nucleotide at the polymorphic site differs from the nucleotide at the polymorphic site of the standard.

According to another aspect, the invention provides a method for determining whether a population of arthropod pests, in particular crustacean pests, in particular copepod pests, in particular sea lice, is resistant towards pyrethroids, comprising the steps of
a) collecting a sample of the organism from its natural environment;
b) isolating mitochondrial genomic material from the collected organism
c) identifying the nucleotide at a polymorphic site in the mitochondrial DNA, the polymorphic site being the site of a single nucleotide polymorphism (SNP) known to be related to resistance towards pyrethroids,
b) comparing the nucleotide polymorphic site of the isolated mitochondrial genomic material with a standard mitochondrial nucleic acid sequence from a member of the species known to be susceptible to pyrethroids, or in the alternative comparing the nucleotide polymorphic site of the isolated mitochondrial genomic material with a standard mitochondrial nucleic acid sequence from a member of the species known to be resistant to pyrethroids, wherein the collected organism is determined to be resistant to pyrethroids if the nucleotide at the polymorphic site differs from the nucleotide at the polymorphic site of the susceptible standard, or is the same as the resistant standard, respectively.

According to another aspect, the invention provides a method for identifying a single nucleotide polymorphism indicative of pyrethroid resistance in an arthropod pest, in particular sea lice, comprising the steps of:
a) determining whether the species to which the organism belongs exhibits incidents of pyrethroid resistance,
b) determining whether said pyrethroid resistance is inherited within the species from the female members to their progeny,
c) in the event said pyrethroid resistance is inherited within the species from the female members to their progeny, then isolating mitochondrial DNA from a member of the species known to be resistant to pyrethroids and comparing said isolated DNA with the mitochondrial DNA of members of the species known to be susceptible to pyrethroids,
d) identifying one or more single nucleotide polymorphisms in the mitochondrial DNA of the resistant member of the species,
e) performing a control to determine whether said single nucleotide polymorphisms are indicative of pyrethroid resistance.

The present invention is in particular based upon the discovery of novel single nucleotide polymorphisms in the mitochondrial DNA of sea lice indicative of resistance to pyrethroids. These particular aspects of the invention, and supporting examples thereof, are describes as follows:
The present disclosure furthermore provides for novel isolated sequences comprising SNPs involved in the resistance towards pyrethroid-based chemotherapy, and their use in determination of pyrethroid resistance in crustaceans. In particular, said method is useful for detection of pyrethroid resistance in copepods, such as sea lice (*Lepeophtheirus salmonis*).

According to one example, the present inventors have identified single nucleotide polymorphisms (SNP) in the mitochondrial genome of a sea lice (*Lepeophtheirus salmonis*), wherein said sea lice is resistant to pyrethroids if at least one of the following nucleotides:
i. C in position 8605;
ii. G in position 9035;
iii. T in position 13957;
iv. G in position 14017;
v. T in position 14065;
or the complementary oligonucleotide thereof, is present in the mitochondrial genome sequence of the one or more sea lice to be analyzed, and wherein the numbering of said positions is in accordance with the sequence depicted in SEQ ID No. 1.

According to yet another aspect, a method is provided, comprising the steps of: a) collecting a sea lice individual from an infested population; b) isolating mitochondrial genomic material from the collected organism; c) determining the nucleotide polymorphic site at the positions 8605, 9035 and 14065 of the isolated mitochondrial DNA compared with of the mitochondrial nucleic acid sequence SEQ ID No. 1, wherein said sea lice is resistant to pyrethroids if at least one of the nucleotides, or a complementary oligonucleotide thereof, is present in position 8605, 9035, 13957, 14017, and 14065, as indicated above respectively. According to one embodiment of the above method, said step c) is performed using a primer selected from the group consisting of SEQ ID No. 6-15.

A method according to yet another embodiment, said step c) is performed using at least one probe selected from the group consisting of SEQ ID No. 16-20. According to yet another embodiment, said step c) comprises nucleic acid amplification, e.g. using polymerase chain reaction.

According to yet another embodiment, said step c) is performed by contacting mitochondrial DNA sequence of the sea lice to be analyzed with a detection reagent, and determining which nucleotide is present in position 8605, 9035, 13957, 14017 and 14065.

According to yet another embodiment, said detection reagent is an oligonucleotide probe.

According to yet another embodiment, said step c) is performed using SNP specific probe hybridization, SNP specific primer extension, SPN specific amplification, sequencing, 5' nuclease digestion, molecular beacon assay, oligonucleotide ligation assay, size analysis, single-stranded conformation polymorphism analysis, denaturing gradient gel electrophoresis.

According to another embodiment of the present method of the invention, the pyrethroid is selected from the group consisting of deltamethrin, and cis-cypermethrin.

According to another embodiment, the present disclosure provides an isolated oligonucleotide sequence comprising small nucleotide polymorphism (SNP) associated with pyrethroid resistance in sea lice, wherein said isolated oligonucleotide sequence comprises nucleotides that distinguishes sea lice which are resistant to pyrethroids from non-resistance to pyrethroids, and wherein the SNP are present in the mitochondrial DNA.

According to one embodiment, the oligonucleotide of the present disclosure comprises a single-nucleotide polymorphism (SNP) associated with pyrethroid resistance in sea lice, wherein said isolated oligonucleotide sequence comprises nucleotides that distinguishes sea lice which are resistant to pyrethroids from non-resistant sea lice, and which is identical or has at least 80% sequence identity with a sequence selected from the group consisting of SEQ ID No. 4and 5 or a fragment thereof, and complementary sequences of SEQ ID No. 4 and 5 and fragments thereof, provided that at least one SNP selected from the group of SNPs consisting of T8605C (U8605C in case of RNA), A9035G, C13957T (U13957T in case of RNA), A14017G, and C14065T (C14065U in case of RNA) is present, and wherein the numbering of said positions is in accordance with the sequence depicted in SEQ ID No. 1.

According to one embodiment, an oligonucleotide probe or oligonucleotide primer is provided comprising an oligonucleotide sequence being homologous to a fragment of an isolated oligonucleotide sequence according to SEQ ID 16, said probe or primer being specific for a mitochondrial DNA sequence of sea lice associated with pyrethroid resistance comprising at least one SNPs selected from the group consisting of T8605C and A9035G of SEQ ID No. 3, and C13957T, A14017G, and C14065T of SEQ ID 4, and wherein the numbering of said positions is in accordance with the sequence depicted in SEQ ID No. 1.

According to yet another embodiment, the probe or primer according to the present invention comprising at least 8 contiguous nucleotides of SEQ ID No. 1, including at least one of the nucleotide C, G, T, G, T or a complementary oligonucleotide thereof in the position corresponding to position 8605, 9035, 13957, 14017 and 14065, respectively, of SEQ ID 1.

According to one embodiment, a probe is provided, wherein the sequence of said probe is selected from the group consisting of SEQ ID No. 16, SEQ ID No. 17, SEQ ID No. 18, SEQ ID No. 19 and SEQ ID No. 20, and sequences having at least 80% sequence identity therewith.

According to one embodiment, a primer is provided, wherein the sequence is selected from the group consisting of SEQ ID No. 6, SEQ ID No. 7, SEQ ID No. 8, SEQ ID No. 9, SEQ ID No. 10, SEQ ID No. 11, SEQ ID No. 12, SEQ ID No. 13 and SEQ ID No. 14, and SEQ ID No. 15, and sequences having at least 80% sequence identity therewith.

Furthermore, the present disclosure provides a kit for detection of pyrethroid resistance in crustaceans, such as copepods, e.g. in sea lice (Lepeophteirus salmonis), comprising a probe or primers according to the present invention.

Finally, the present disclosure provides an isolated oligonucleotide sequence comprising at least 8 contiguous nucleotides of the sequence selected from the group consisting of SEQ ID No. 4 and SEQ ID No. 5, and wherein said sequence comprises at least one of the nucleotide C, G, T, G, T or U or a complementary oligonucleotide thereof in the position corresponding to position 8605, 9035 and 14065, and wherein the numbering of said positions is in accordance with the sequence depicted in SEQ ID No. 1.

### Figures

**Figure 1** illustrates the hybridization experiment showing that pyrethroid resistance characteristics are transmitted from female sea lice to their progenies.
**Figure 2** illustrates the experiment flow of the hybridization experiment. Two field-collected strains of lice were initially used to create two hybrid strains: *Ls*HybridG with *Ls*Gulen as its maternal strain, and *Ls*HybridV with *Ls*Vikna as its maternal strain.

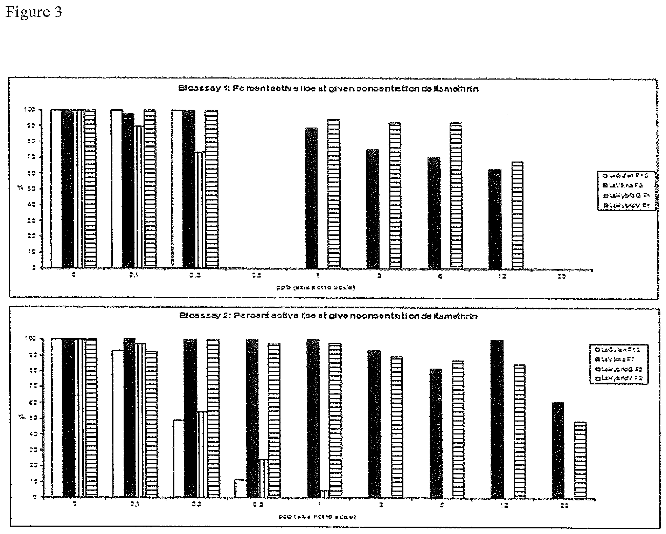

**Figure 3** show percent active lice in deltamethrin bioassays 1 and 2. In bioassay 1 the concentrations 0.5 and 20 ppb were not included, and in bioassay 2 LsGulen F7 was not tested for 20 ppb.

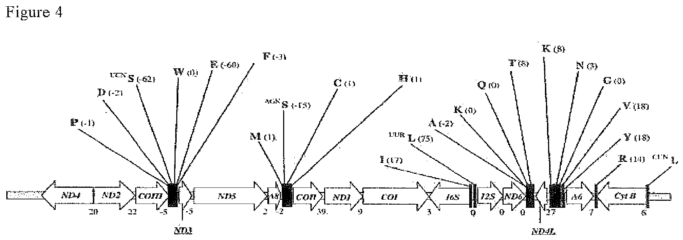

**Figure 4** shows the organization of mitochondrial DNA of *L. salmonis.*

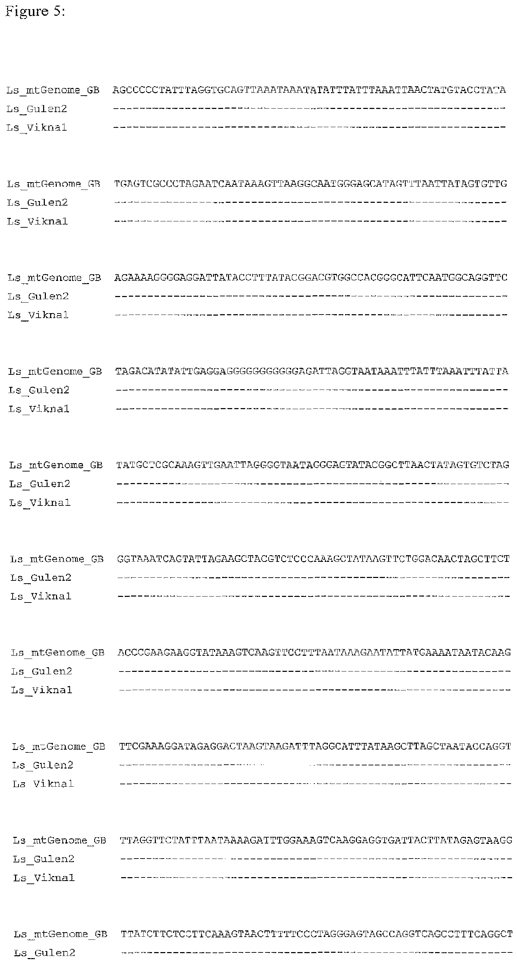

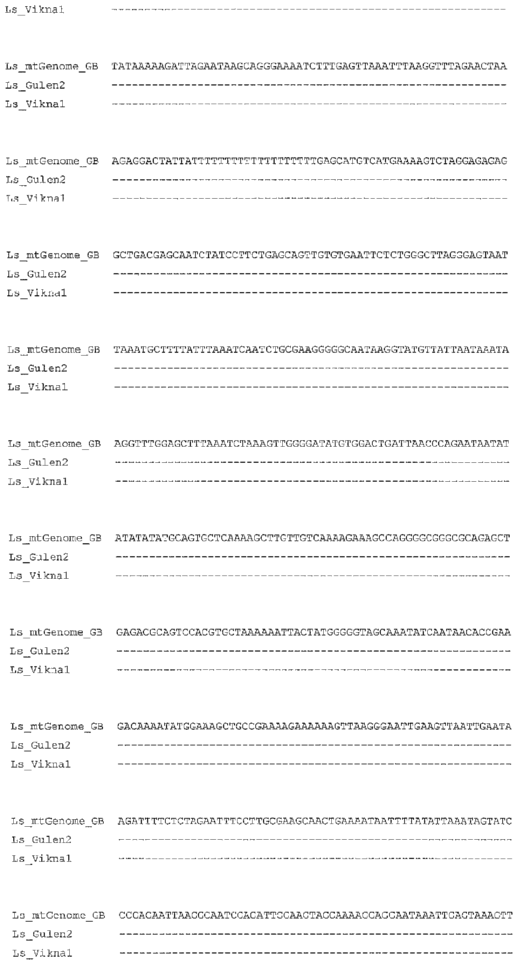

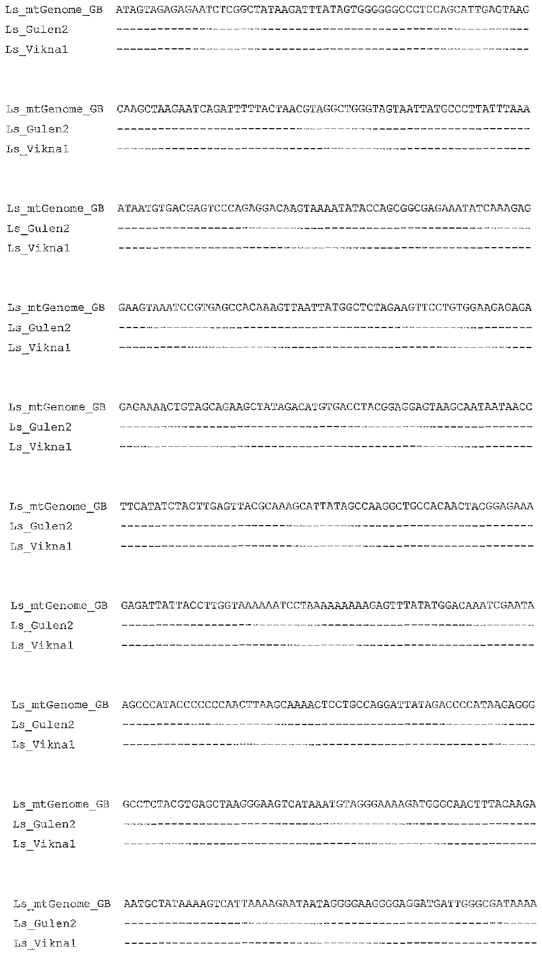

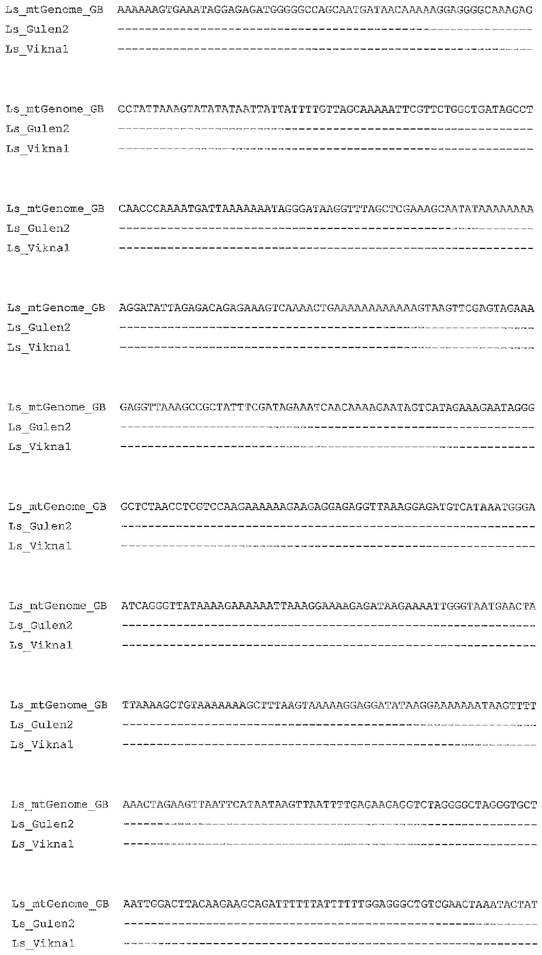

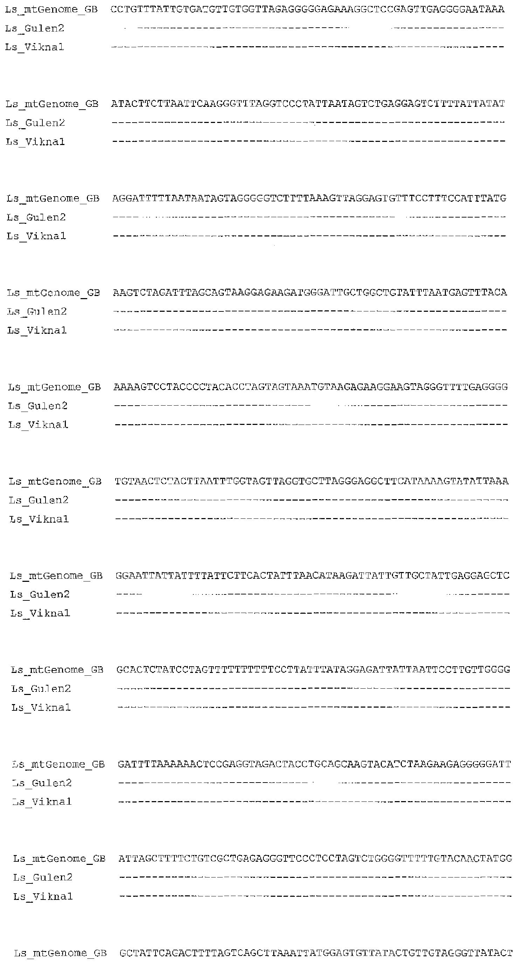

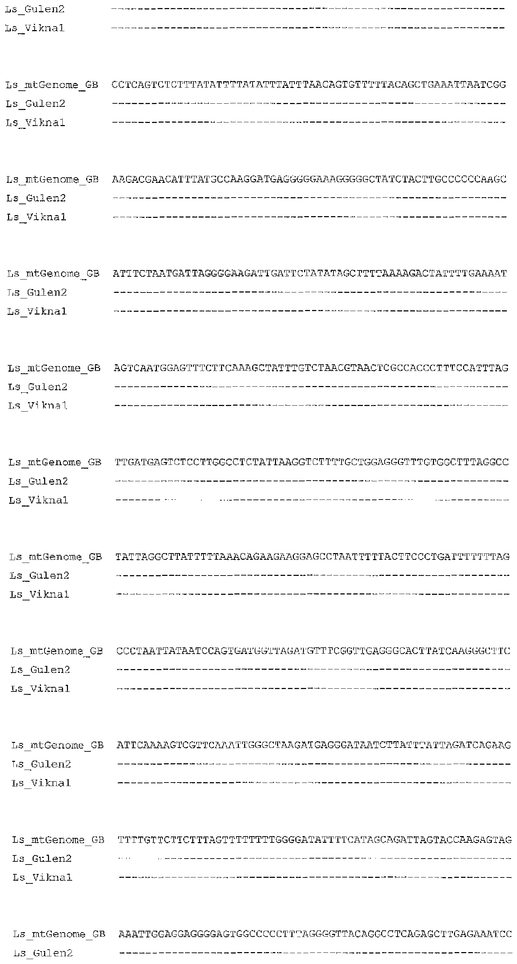

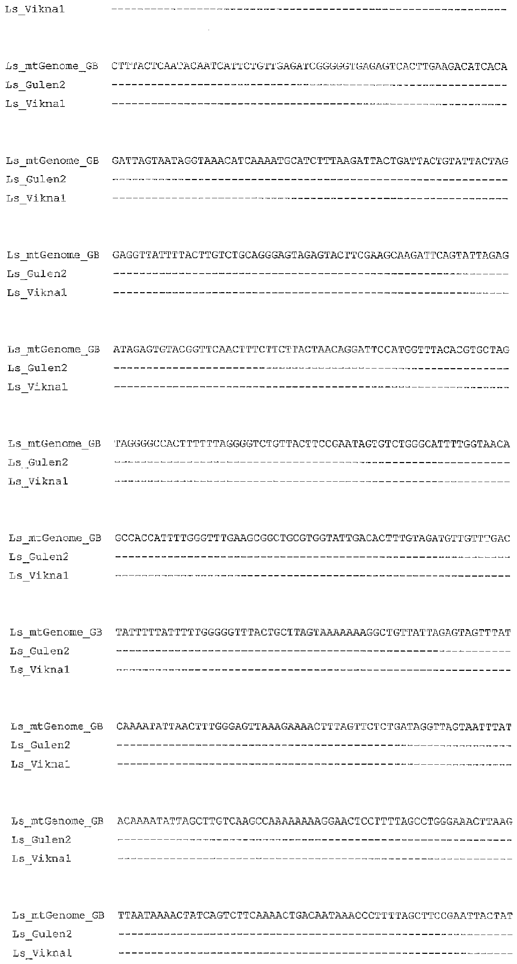

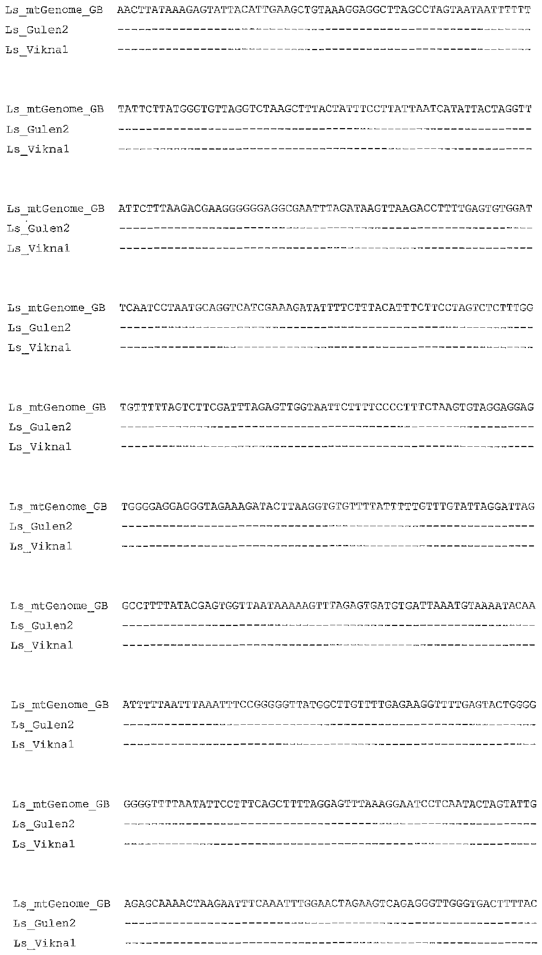

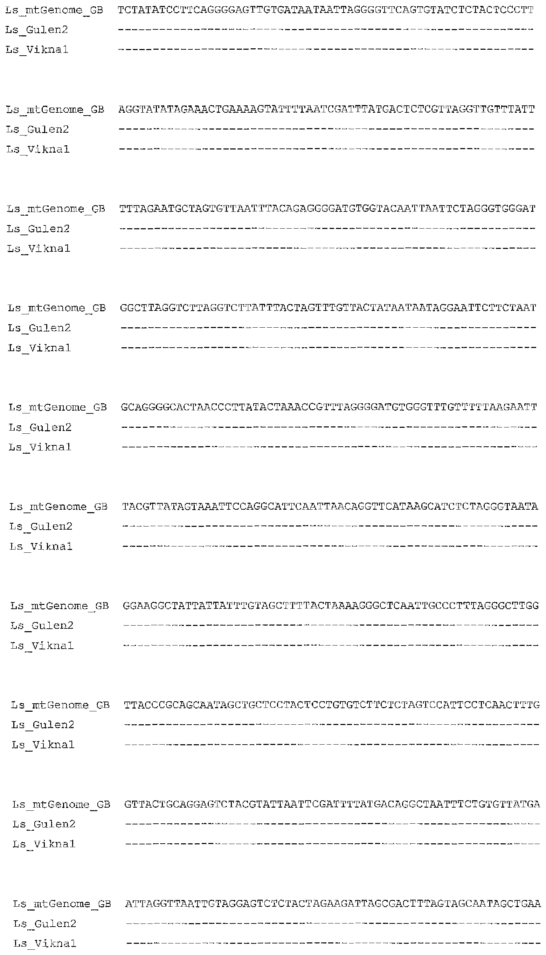

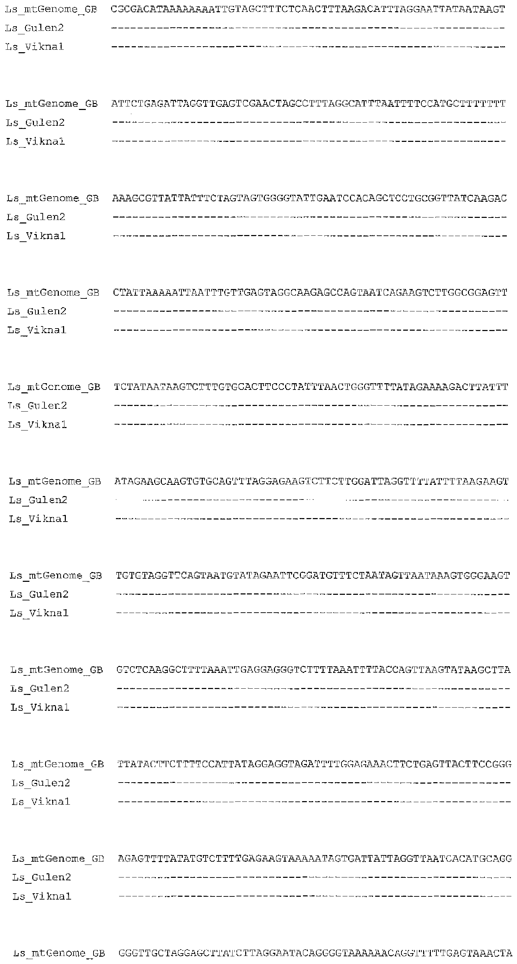

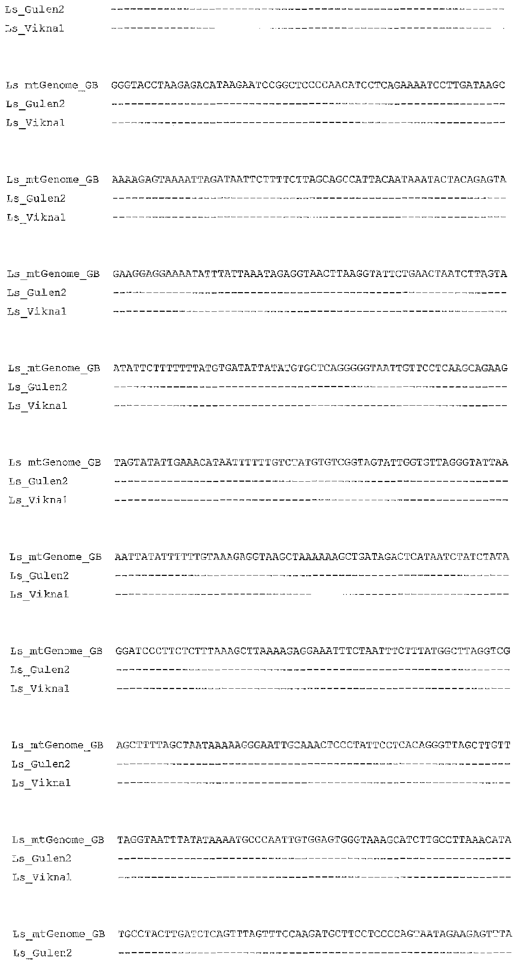

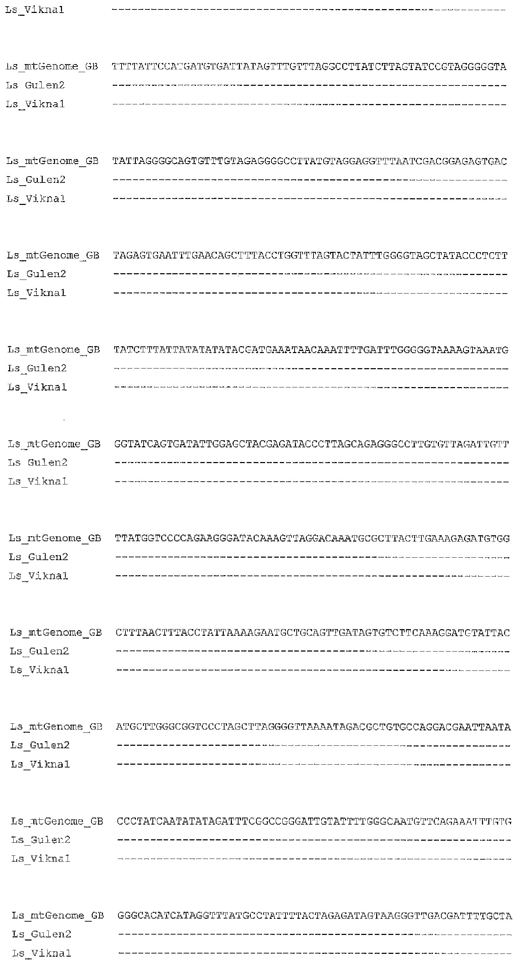

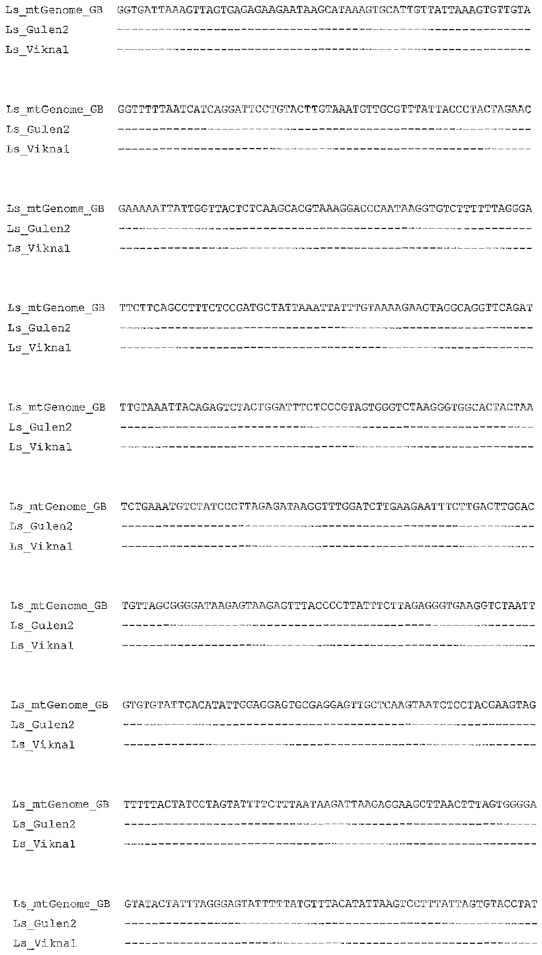

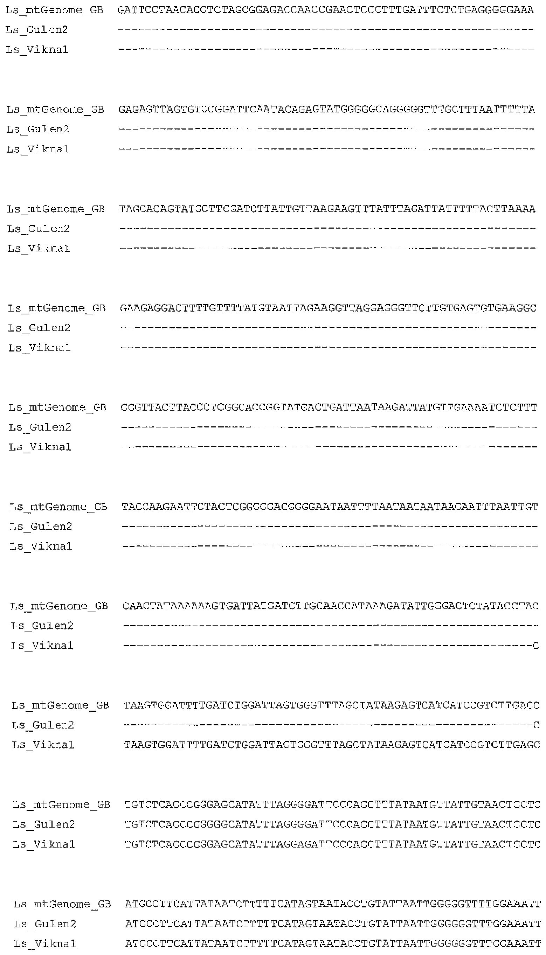

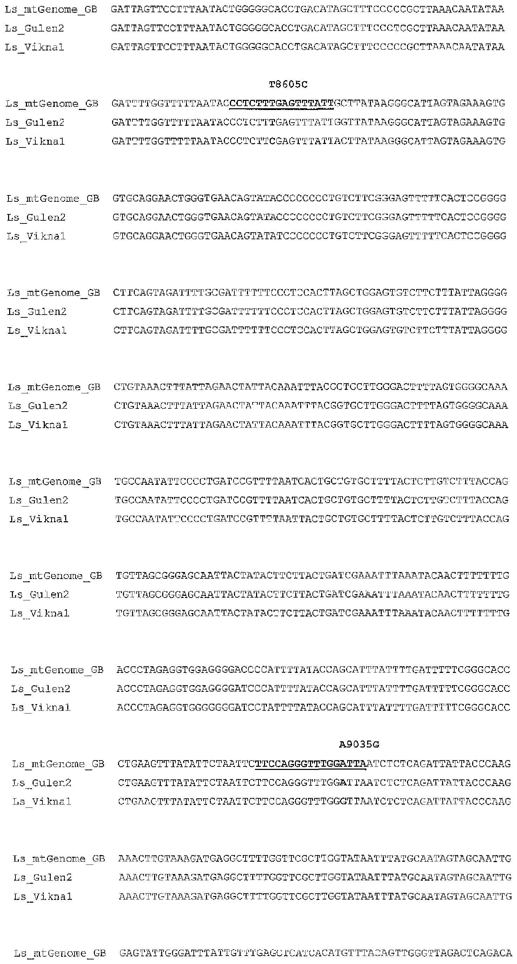

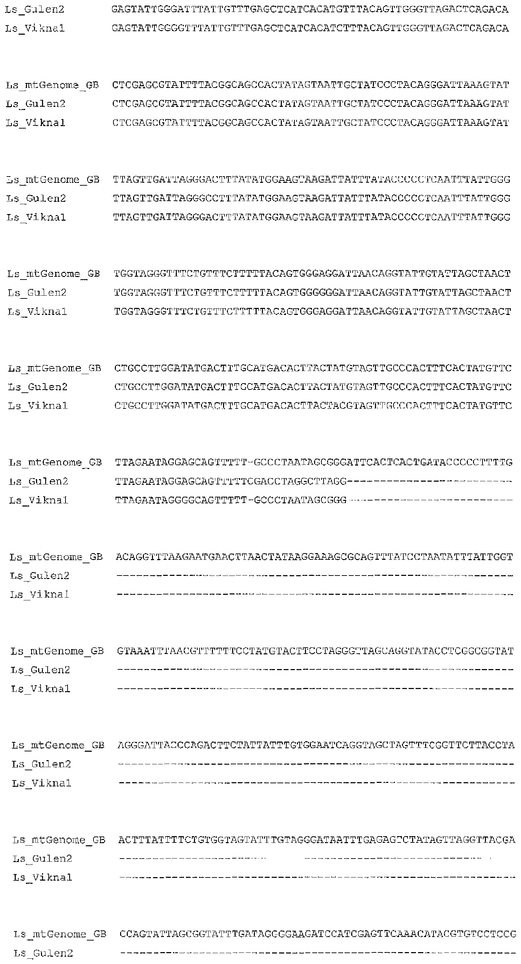

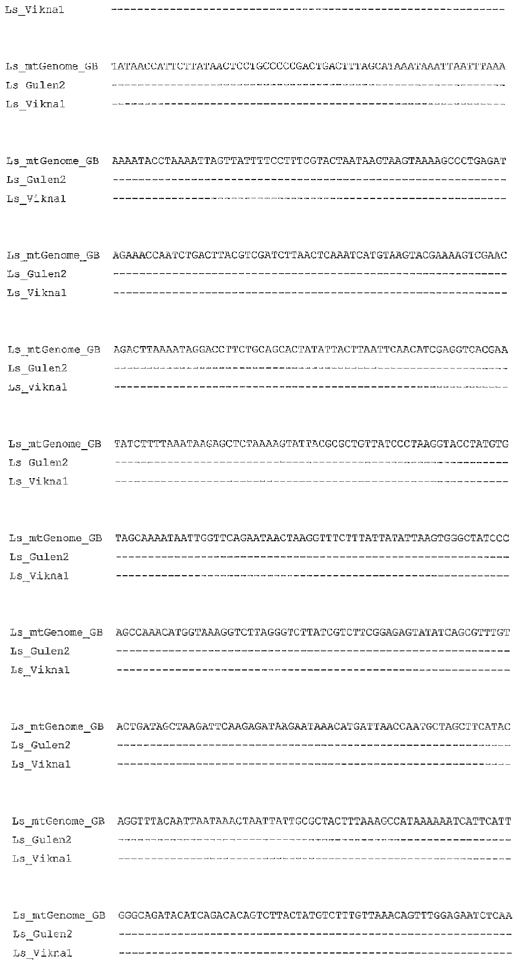

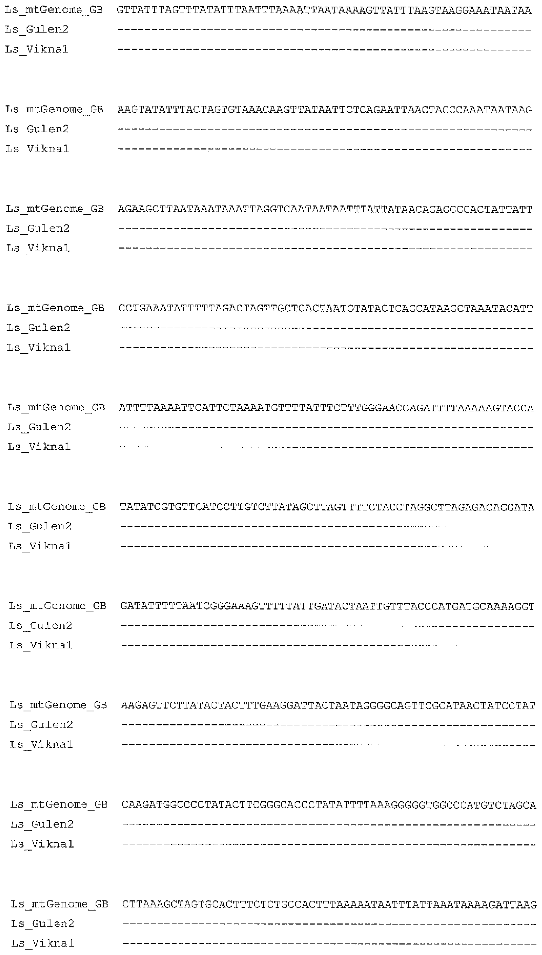

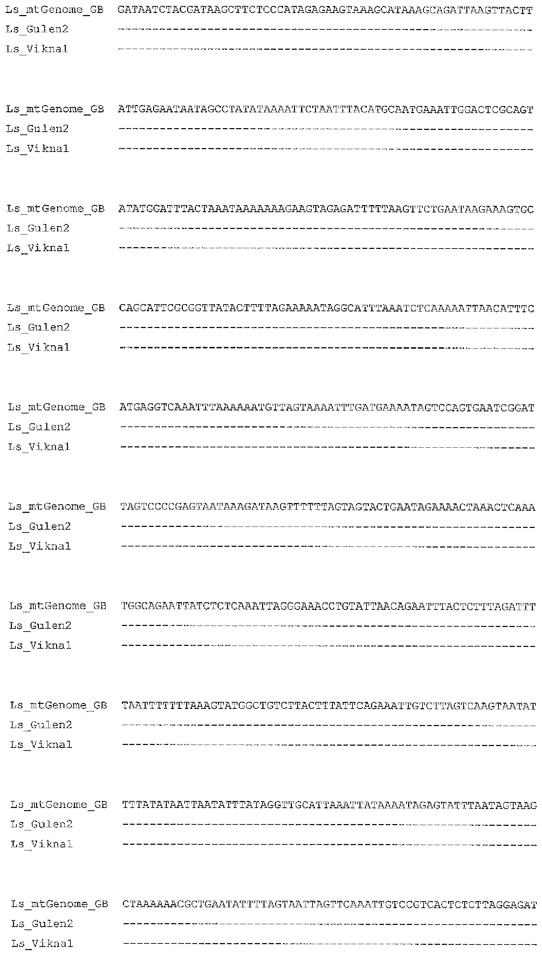

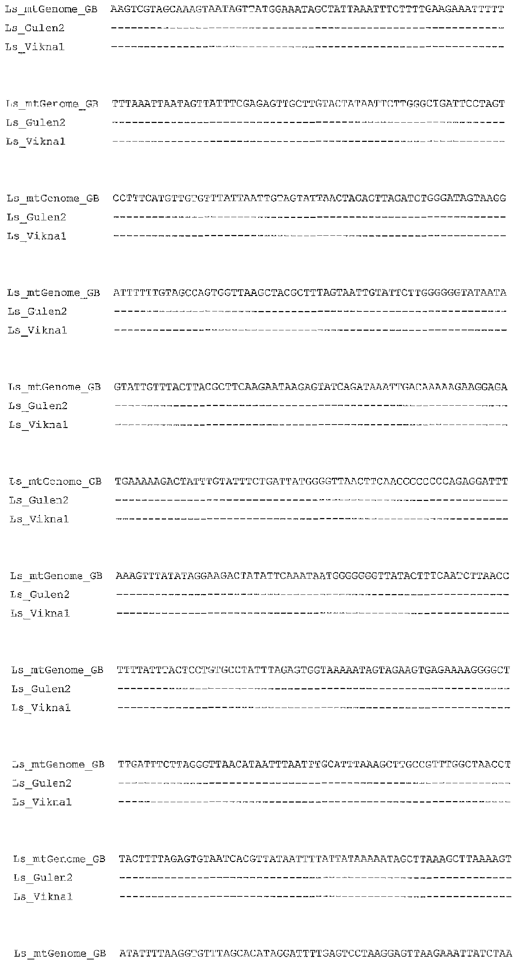

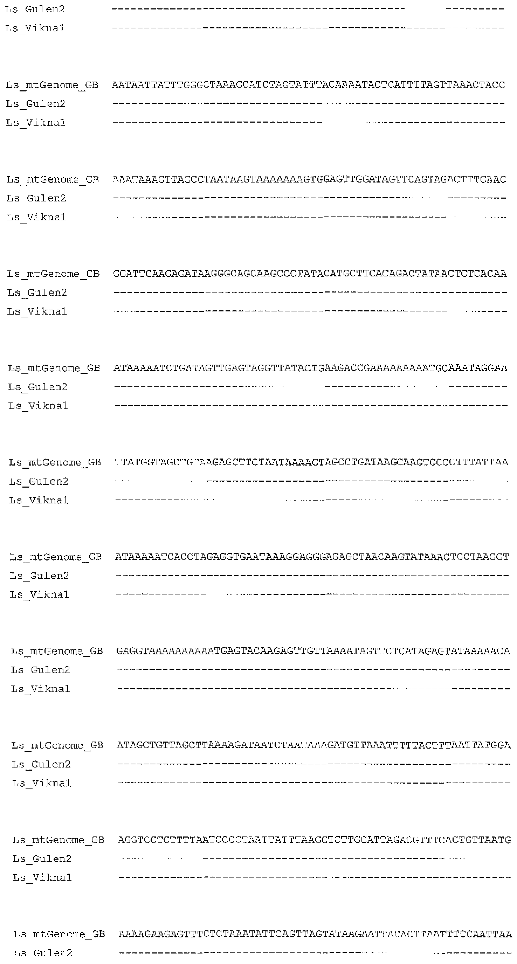

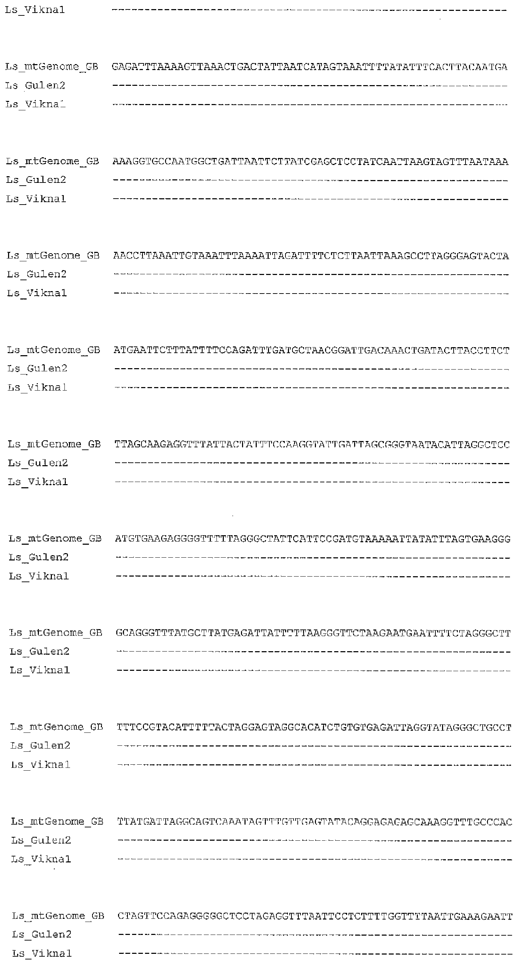

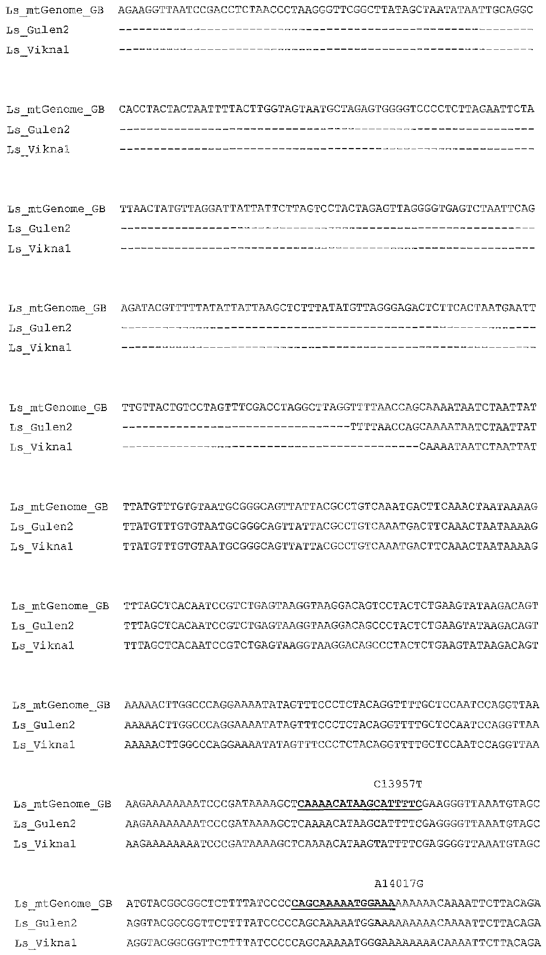

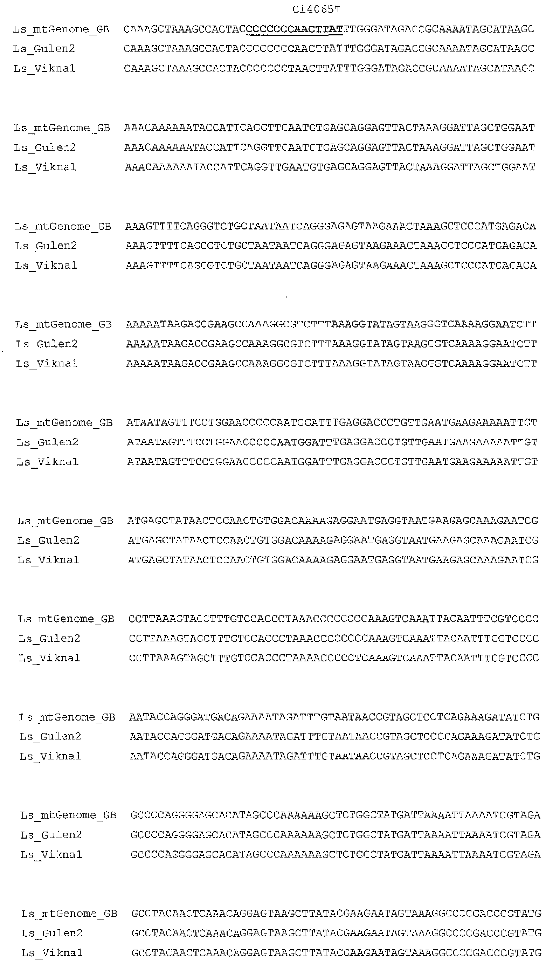

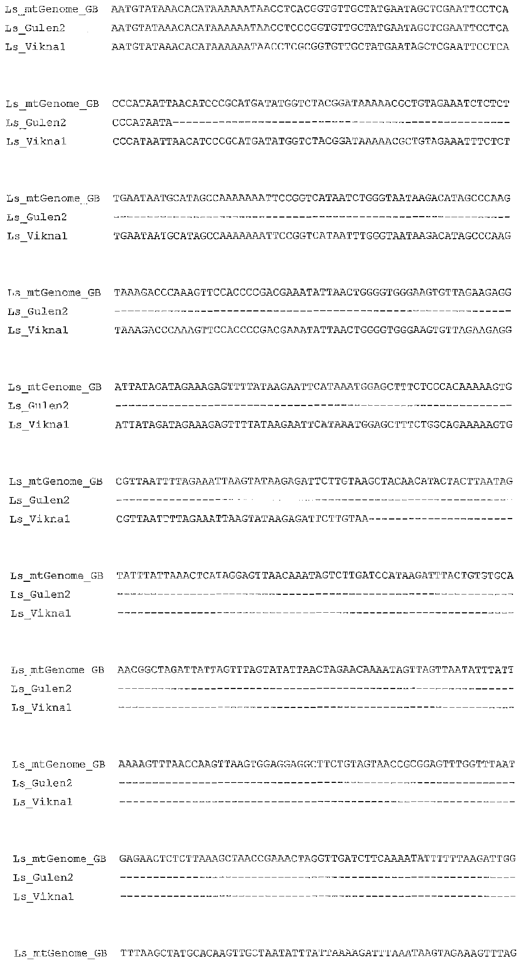

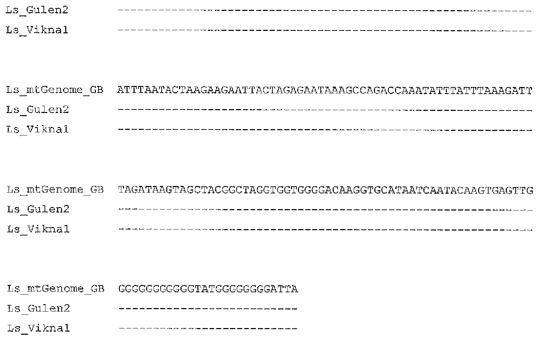

**Figur 5** shows an alignment of mitochondria sequences from *L*. *salmonis.* The upper sequence is the reference sequence of the *L*. *salmonis* mtDNA deposited by Tjensvoll et al (2005) with NCBI accession number NC_007215 (http://www.ncbi.nim,nlm.gov/nuccore/NC 007215.1), corresponding to SEQ ID No. 1. The start point in this sequence is in the D-loop (i.e. the non-coding region of the mtDNA). The sequence named LsGulen2 is isolated from a pyrethroid sensitive strain and is depicted in SEQ ID No. 2 and 3. The sequence named LsVikna1 is isolated from a strain resistant to pyrethroids, and is depicted in SEQ ID No. 4 and 5.

The present invention and its various embodiments will be described in more detail in the following.

### Detailed description of the invention

The present invention provides an in vitro method for determination of pyrethroid resistance in arthropods, in particular crustaceans, including copepods, in particular sea lice such as *Lepeophtheirus salmonis* and novel SNPs, based on the surprising findings that mutations linked with resistance against pyrethroid in sea lice was found in mitochondrial DNA.

Although pyrethroid resistance linked SNPs presented in the experimental data was identified in the sea lice species *Lepeophtheirus salmonis,* the skilled person will acknowledge, based on the teaching herein, that the present method and the present oligonucleotides might be used to determine pyrethroid resistance is crustaceans, in particular copepods, in particular copepods belonging to the family Caligidae. According to one embodiment, the present method and present oligonucleotides are useful for detection of pyrethroid resistance in copepod selected from the group consisting of *Lepeophteirus salmonis, Caligus elongatus,* and *Caligus rogercresseyi.*

Throughout the application, the term "sea lice" is to be understood to mean any copepod belonging to the family Caligidae. However, whenever the term "sea lice" is used in connection with the experimental data in the present application, sea lice refers to the specie *Lepeophtheirus salmonis.*

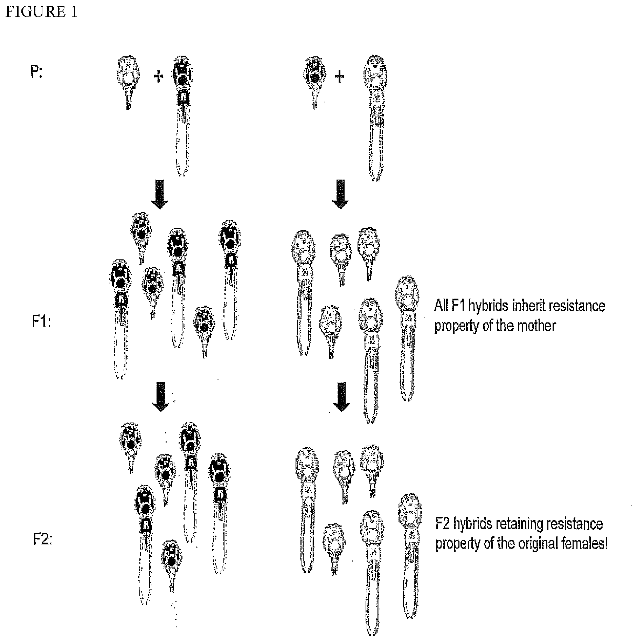

By hybridization of adult male sea lice shown to be sensitive to pyrethroids with preadult female sea lice shown to be resistant towards pyrethroids, the present inventors where able to show that the resistant female sea lice transmitted their pyrethroid resistant characteristics to their progenies (cf. figure 1, example 1). Resistant adult male sea lice did not transfer their pyrethroid resistant characteristics when crossed with sensitive females. The mechanism of action could therefore be linked to the mitochondrion of the sea louse. Based on further extensive sequence analysis of mitochondrial genomes of pyrethroid resistant sea lice, and comparison with the mitochondrial genomic sequence of a known reference strain (GenBank acc. No. NC_007215 (Tjensvoll et al., 2005, Gene, 353 (2), 218-230), SEQ ID No. 1) and mitochondrial sequences of a pyrethroid sensitive sea lice strain (SEQ ID No. 2 and 3), the present inventors have identified five single nucleotide polymorphism sites that are clearly linked to pyrethroid resistance (figure 5) in sea lice.

More particularly, sequences from 180 individual *L*. *salmonis* obtained from 6 different locations (see Tjensvoll et al 2006, *supra*) from Col and cyt b were compared to sequences from pyrethroid sensitive and resistant lice and the newly identified SNPs characteristic for resistant strains are only present in the resistant populations. Two of the SNPs uniquely associated with resistance to pyrethroids are found in the Col gene, more specifically in position 8605 and position 9035 (T8605C, A9035G). Three SNPs found to be uniquely associated with resistance to pyrethroids is found in the cyt b gene, more specifically in position 13957 (C13957T), 14017 (A14017G), and 14065 (C14065T).

The start point of the mitochondrial DNA sequence of SEQ ID No. 1 is in the D-loop (i.e. the non-coding region of the mtDNA). The numbering of the positions of the identified SNPs are based of the mitochondrial sequence of Tjensvoll et al, 2005, supra, deposited with the GenBank Acc. No. NC_007215 as depicted in SEQ ID No. 1. It is to be understood that whenever referring to the positions of the SNPs identified according to the present invention, the numbering is throughout the present description made according to the numbering of the reference strain sequence (GenBank Acc. No. NC_007215) if not otherwise stated.

Based on the identification of the five SNPs responsible for pyrethroid resistance in sea lice, a method for determination of pyrethroid resistance in crustaceans, such as copepods, in particular in sea lice, e.g. *Lepeophtheirus salmonis* PRLS have been provided. More particular, a method for detection of PRLS is provided comprising the steps of detecting single nucleotide polymorphism (SNP) associated with pyrethroid resistance in the mitochondrial DNA of a sea lice to be analyzed, wherein said sea lice is resistant to pyrethroids if at least one of the following nucleotides:
i. C in position 8605;
ii. G in position 9035;
iii. T in position 13957;
iv. G in position 14017;
v. T in position 14065;
or the complementary oligonucleotide thereof,
is present in the mitochondrial DNA sequence of the sea lice, and wherein the numbering of said positions is in accordance with the sequence depicted in SEQ ID No. 1.

In addition to the qualitative determination of the SNPs involved in pyrethroid resistance according to the present invention, the present invention also provides for a method for gradation of pest population being susceptible of developing resistance, i.e. taking into account the composition of haplotypes that are determined within a pest population (see also example 3 for practical details).

According to the present invention, "single-nucleotide polymorphisms (SNP)" is to be understood to refer to a nucleotide sequence variation occurring when a singe nucleotide, A, T (U), C or G in the genome, or other shared sequences (e.g. RNA) or fragments thereof, differs between members of a biological species, such as between variants of *Lepeophtheirus salmonis.* SNPs may fall within coding sequences of genes, or non-coding regions of genes, or in the regions between the genes in a genome. The five SNPs identified according to the present invention fall within the genes encoding the cytochrome oxidase subunit I (Col) and cytochrome B (Cytb), respectively.

Furthermore, as used herein, an "oligonucleotide sequence" or "nucleic acid sequence" is generally an oligonucleotide sequence or a nucleic acid sequence containing a SNP described herein, or one that hybridizes to such molecule such as a nucleic acid sequence with a complementary sequence.

The skilled person is well aware of the fact that nucleic acid molecules may be double-stranded or single-stranded, and that reference to a particular site of one strand refers, as well, to the corresponding site on a complementary strand. Thus, when defining a SNP position, reference to an adenine (A), a thymine (T) (uridine (U)), a cytosine (C) or a guanine (G) at a particular site on one strand of a nucleic acid is also to be understood to define a thymine (uridine), adenine, guanine, or cytosine, respectively, at the corresponding site on a complementary strand of the nucleic acid molecule. Thus, reference may be made to either strand in order to refer to a particular SNP position. The oligonucleotide probes and oligonucleotide primers according to the present invention may be designed to hybridize to either strand, and SNP detection methods disclosed herein may thus also in general target either strand.

An "isolated nucleic acid" as used herein is generally one that contains at least one of the SNPs described herein or one that hybridizes to such molecule, e.g. a nucleic acid with a complementary sequence, and is separated from most other nucleic acids present in the natural source of the nucleic acid, and is thus substantially free of other cellular material.

### Oligonucleotide probes and oligonucleotide primers

The present disclosure provides oligonucleotide probes and oligonucleotide primers that may be used for detection of the presence of the SNPs according to the present invention in mitochondrial DNA of a sea lice to be tested, and thus for determination of pyrethroid resistance. The detection of nucleic acids present in a biological sample is widely applied in both human and veterinary diagnosis, wherein nucleic acids from e.g. pathogens present in biological samples are isolated and hybridized to one or more hybridizing probes or primers are used in order to amplify a target sequence.

One or more oligonucleotide probes may be constructed based on the teaching herein and used in hybridization based detection methods where upon the binding of the oligonucleotides to the target sequence enables detection of the presence of at least one of the SNPs described herein if present in the sample to be tested. The skilled person will acknowledge that an oligonucleotide probe according to the present disclosure may be a fragment of DNA or RNA of variable length used herein in order to detect an SNP in a target sequence, e.g. single-stranded mitochondrial DNA or RNA, upon hybridization of the oligonucleotide probe to complementary sequence(s) of the said target sequence to be analyzed. The oligonucleotide probe according to the present disclosure may furthermore be labeled with a molecular marker in order to easily visualize that hybridization, and thus detection of the SNPs disclosed herein, have been achieved. Molecular markers commonly known to the skilled person may be used, e.g. a radiolabel, and more preferably, a luminescent molecule or a fluorescent molecule enabling the visualisation of the binding of the probe(s) to a target sequence.

A oligonucleotide probe according to the present disclosure is able to hybridize to another nucleic acid molecule, such as the single strand of mitochondrial DNA or RNA originating from a sea lice to be analysed, under appropriate conditions of temperature and solution ionic strength, cf. e.g. Sambrook et al., Molecular Cloning: A laboratory Manual (third edition), 2001, CSHL Press, (ISBN 978-087969577-4). The condition of temperature and ionic strength determine what the skilled person will recognise as the "stringency" of the hybridization. The suitable stringency for hybridisation of a probe to target nucleic acids depends on inter alia the length of the probe and the degree of complementation, variables well known to the skilled person. A oligonucleotide probe according to the present invention typically comprises a nucleotide sequence which under stringent conditions hybridize to at least 8, 10, 12, 16, 20, 22, 25, 30, 40, 50 (or any other number in-between) or more consecutive nucleotides in a target nucleic acid molecule, e.g. single-stranded mitochondrial DNA or RNA isolated from the sea lice to be analyzed according to the present invention. According to one embodiment, the oligonucleotide probe according to the present invention comprises about 13 to 25 consecutive nucleotides. It is to be understood that the oligonucleotide probe according one embodiment comprise one of the SNPs described herein or the complement thereof. New technology like specific Locked Nucleic Acid (LNA) hybridization probes allows for the use of extremely short oligonucleotide probes (You Y.; Moreira B.G.; Behlke M.A. and Owczarzy R. (2006). "Design of LNA probes that improve mismatch discrimination". Nucleic Acids Res. 34 (8): e60. doi:10.1093/nar/gkl175. PMC 1456327. PMID 16670427.) According to one embodiment, probes are provided which are selected from the group consisting of SEQ ID No. 16-20.

The present disclosure furthermore provides oligonucleotide primers useful for amplification of any given region of a nucleotide sequence, in particular a region containing one of the SNPs described herein. An oligonucleotide primer according to the present invention typically comprises a nucleotide sequence at least 8, 10, 12, 16, 20, 22, 25, 30, 40, 50 (or any other number in-between) or more consecutive nucleotides. According to one embodiment, the oligonucleotide primer according to the present invention comprises about 18-25 consecutive nucleotides, more preferably about 20 nucleotides.

As used herein, the term "oligonucleotide primer" is to be understood to refer to a nucleic acid sequence suitable for directing an activity to a region of a nucleic acid, e.g. for amplification of a target nucleic acid sequence by polymerase chain reaction (PCR). According to one embodiment of the present disclosure "oligonucleotide primer pairs" is provided suitable for amplification of a region of mitochondrial genome material comprising the SNPs according to the present invention.

The skilled person will acknowledge that an oligonucleotide primer according to the present disclosure may be a fragment of DNA or RNA of variable length used herein in order to detect an SNP in a target sequence, e.g. single-stranded mitochondrial DNA or RNA, upon alignment of the oligonucleotide probe to complementary sequence(s) of the said target sequence to be analyzed. An oligonucleotide primer according to the present invention may furthermore be labeled with a molecular marker in order to enable visualization of the results obtained. Various molecular markers or labels are available, dependent on the SNP detection method used.

An oligonucleotide primer according to the present disclosure typically comprises the appropriate number of nucleotides allowing that said primer align with the target sequence to be analyzed. It is to be understood that the oligonucleotide primer according to the present invention according to one embodiment comprises the SNP described herein or the complement thereof. According to one embodiment, the probes useful in order to determine pyrethroid resistant sea lice is selected from the group consisting of SEQ ID No. 16, SEQ ID No.17, SEQ ID No.18, SEQ ID No.19, and SEQ ID No.20.

According to one embodiment of the present disclosure primer pairs are provided selected from the group consisting of SEQ ID No. 6 - SEQ ID No. 15 (see also table 4 below).

Oligonucleotide probes and oligonucleotide primers according to the present disclosure may be synthesized according to methods well known to the skilled person.

The present disclosure furthermore relates to isolated nucleic acid sequences and variants or fragments thereof having at least 70% identity with the nucleic acid sequences depicted in SEQ ID NO. 4, or SEQ ID No. 5, or fragments thereof. The term "% identity" is to be understood to refer to the percentage of nucleotides that two or more sequences or fragments thereof contains, that are the same. A specified percentage of nucleotides can be referred to as e.g. 70% identity, 80% identity, 85% identity, 90% identity, 95% identity, 99% identity or more (or any number in between) over a specified region when compared and aligned for maximum correspondence. The skilled person will acknowledge that various means for comparing sequences are available. For example, one non-limiting example of a useful computer homology or identity program useful for determining the percent homology between sequences includes the Basic Local Alignment Search Tool (BLAST) (Altschul et al., 1990, J. of Molec. Biol., 215:403-410, "The BLAST Algorithm; Altschul et al., 1997, Nuc. Acids Res. 25:3389-3402, , Karlin and Altschul 1990, Proc. Nat'l Acad. Sci. USA, 87:2264-68; 1993, Proc. Nat'l Acad. Sci. USA 90:5873-77).

### SNP identification methods

Upon the identification of the SNPs associated with pyrethroid resistance in a pest according to the present invention, the skilled person will acknowledge that various methods commonly used in order to detect polymorphisms within a population of pests may be used. Both methods based on genome sequencing, hybridization and enzyme based methods are applicable for determining whether a pest is pyrethroid resistant in accordance with the present inventions.

Various enzyme based methods are available for the skilled person, of which a number of polymerase chain reaction (PCR) based methods are available. For example, Perkin Elmer Life Sciences provides SNP detection kit that may be used in order to determine whether a sea louse is pyrethroid resistant (AcycloPrime^{™}-FP SNP Detection). In said method, a thermostable polymerase is used which extends an oligonucleotide primer according to the present invention by one base, then ending the oligonucleotide primer one nucleotide immediately upstream of the relevant SNP position by the incorporation of fluorescent dye-labeled terminators. The identity of the base added is then determined by the increase fluorescence polarization of its linked dye (http://shop.perkinelmer.com/Content/Manuals/MAN AcycloPrimeSNPKit.pdf). Oligonucleotide primers according to the present invention useful in such a method would thus be constructed in order to facilitate the extension of the primer by one base in the position selected from the group 8605, 9035, 13957, 14017, or 14064, relative to SEQ ID No. 1 in the case where the pest is sea lice.

Another enzyme based method that may be used is restriction fragment length polymorphism (RLFP), utilizing that various, highly specific endonuclease upon digestion of the target sample, i.e. mitochondrial genome, results in different fragments that may be separated by gel electrophoresis.

Yet another enzyme based method that may be utilized in accordance with the present invention is the flap endocuclease (FEN) method. In said method, a structure-specific endonuclease is used to cleave a three-dimensional complex formed by hybridization with the target DNA, e.g. mitochondrial genomic material from sea lice, and where annealing with a target sequence comprising the SNP of interest triggers cleavage by the endonuclease (Oliver, 2005, Mutat. Res., vol. 573 (1-2), pp. 103-110).

Yet another method applicable in respect of the present invention is based on the use of TaqMan® Assays (Invitrogen). In said assay the oligonucleotide primers used in order to detect an SNP is labeled in both the 5'- and the 3' end, i.e. with a fluorophore at the 5'end of the oligonucleotide primer, and a quencher at the 3'-end of the oligonucleotide primer. Upon annealing of the oligonucleotide primer with a target sequence, the Taq polymerase will extend the oligonucleotide primer and form a nascent strand, followed by degradation of the oligonucleotide primer being annealed to the target, said degradation eventually resulting in the release of the fluorophore and provide a cleavage close to the quencher. The fluorescence signal produced is proportional to the fluorophore released. Various fluorophore labels may be used, such as e.g. 6-carboxyfluorescein, tetrafluorofluorescein. As quenchers, tetramethylrhodamine or dihydrocyclopyrroloindol may be used.

Several hybridization methods for detection of SNPs are available to the skilled person, and which may be utilized in accordance with the method of the present invention. For example, the SNPs according to the present invention may be detected utilizing molecular beacon technology. According to this aspect of the present invention, oligonucleotide primers may be synthesized comprising complementary regions at each end allowing the formation of a hairpin loop, and wherein a fluorophore is attached at one end of the oligonucleotide primer, and a quenching agent is attached to the other end, and wherein fluorescence signal is produced upon binding to a DNA target of interest, i.e. mitochondrial genomic material isolated from the sea louse to be analyzed.

Yet another method applicable in respect of the present invention is based on DNA or RNA sequencing, which is the process of determining the precise order of nucleotides within a molecule. It includes any method or technology that is used to determine the order of the four bases (adenine, guanine, cytosine, and thymine) in a strand of DNA. The skilled person is well known with the various commonly known DNA and RNA sequencing methods that may be used according to the present invention, such as e.g. shotgun sequencing or bridge PCR sequencing.

### Isolation of sea lice genomic material

The method according to the present invention may according to one embodiment involve the isolation of a biological sample from a sea lice and testing for the presence of a SNP associated with PRLS in the mitochondrial genome. The skilled person will acknowledge that the SNPs identified according to the present invention may be detected by analyzing mitochondrial DNA as well as mitochondrial RNA, dependent upon the detection method used.

In order to determine whether a sea louse is pyrethroid resistant in accordance with the present invention, mitochondrial genomic material may be isolated. Various methods for obtaining genomic material well known to the skilled person are available. The skilled person will acknowledge that any tissue (i.e. any part of the sea lice) may be used in order to extract mitochondrial genomic material. Furthermore, the mitochondrial genomic material to be analyzed according to the present invention may be obtained from sea lice of any life stages, e.g. the free swimming stages (nauplius stage I and II), the copepod stage, the pre-adult (chalimus stages 1-4), or the adult stage (adult male or adult female). According to one embodiment, tissue removed from sea lice to be tested is maintained in 70% ethanol or other conservation liquid prior to further isolation of genomic material. DNA may be extracted from the obtained tissue using commonly available DNA extraction/isolation methods, such as e.g. DNeasy DNA Tissue Kit according to the protocol of the manufacturer (http://lycofs01.lycoming.edu/∼gcat-seek/protocols/DNeasy_Blood_&_Tissue_Handbook.pdf).

### SNP detection Kits

Based on the teaching herein, the skilled person will acknowledge that, based on the identified SNPs and associated sequence information disclosed herein, detection reagents can be developed and used to determine any SNP described herein individually or in combination, and that such detection reagents can be readily incorporated into kits used for SNP detection known in the art. The term "kit" as used herein in the context of SNP detection reagents are intended to cover e.g. combinations of multiple SNP detection reagents, or one or more SNP detection reagents, such as oligonucleotide probe(s) and oligonucleotide primer(s) or primer sets, arrays/microarrays of nucleic acid molecules, and beads that contain one ore more oligonucleotide probe(s), oligonucleotide primer(s) or other detection reagents useful in the method of the present invention. It is furthermore to be understood that the SNP detection reagents in a kit according to the present invention may furthermore include other components commonly included in such kits, e.g. such as various types of biochemical reagents (buffers, DNA polymerase, ligase, deoxynucleotide triphosphates for chain extension/amplification, etc.), containers, packages, substrates to which SNP detection reagents are attached., etc. necessary to carry the method according to the present invention. According to one embodiment of the present disclosure a kit is provided which comprises the necessary reagents to carry out one or more assays in order to detect the SNP disclosed herein according to the method of the present invention. A kit according to the present disclosure may preferably comprise one or more oligonucleotide probes that hybridize to a nucleic acid target molecule (i.e. mitochondrial genetic material) enabling detection of each target SNP position if present in the material analyzed. Multiple pairs of probes may be included in the kit to simultaneously analyze for the presence of the SNP disclosed herein at the same time. The probes contained in the kit according to the present invention may according to one embodiment be immobilized on a carrier, such as e.g. an array or a bead.

According to one embodiment, the oligonucleotide probes are suitable for the detection of the SNP T8605C. According to another embodiment, the oligonucleotide probes are suitable for the detection of the SNP A9035G. According to yet another embodiment, the oligonucleotide probes is suitable for the detection of the SNP C13957T. According to yet another embodiment, the oligonucleotide probes is suitable for the detection of the SNP A14017G. According to yet another embodiment, the oligonucleotide probes is suitable for the detection of the SNP C14065T. According to yet another embodiment, the kit according to the present invention comprises oligonucleotide probes suitable for detection of all the SNPs described herein.

According to one embodiment, a kit according to the present disclosure comprises oligonucleotide primer(s) and optionally further SNP detection reagents useful in SNP detection methods utilizing oligonucleotide primers or primer pair(s). According to one embodiment, the kit according to the present disclosure comprises a forward primer and a reverse primer for amplifying a region containing a SNP selected from SNP selected from the group of SNPs consisting of T8605C, (U8605C in case of RNA), A9035G, C13957T (or U13957T in case of RNA), A14017G, or C14065T (C14065U in case of RNA). Said kit may furthermore optionally comprise further SNP detection reagents (enzymes and nucleotide triphosphates) necessary for conducting PCR or real time PCR. According to one embodiment, the primer pairs are suitable for the detection of the SNP T8605C. According to another embodiment, the primer pairs are suitable for the detection of the SNP A9035G. According to yet another embodiment, the primer pairs is suitable for the detection of the SNP C13957T. According to yet another embodiment, the primer pairs is suitable for the detection of the SNP A14017G. According to yet another embodiment, the primer pairs is suitable for the detection of the SNP C14065T. According to yet another embodiment, the kit according to the present disclosure comprises primer pairs suitable for detection of all the SNPs described herein.

**Table 1 Sequences related to the invention**

| SEQ ID No. | Description of sequence |
|---|---|
| 1 | Mitochondrial DNA of Lepeophtheirus salmonis from sea lice (GenBank acc. No. NC_007215 (Tjensvoll et al., 2005, Gene, 353 (2), 218-230) |
| 2 | Mitochondrial DNA of Lepeophtheirus salmonis isolated from pyrethroid sensitive strain (Ls_Gulen2 CoI) |
| 3 | Mitochondrial DNA of Lepeophtheirus salmonis isolated from pyrethroid sensitive strain (Ls_Gulen2 Cytb) |
| 4 | Mitochondrial DNA of Lepeophtheirus salmonis isolated from pyrethroid resistant strain (Ls_Viknal CoI) |
| 5 | Mitochondrial DNA of Lepeophtheirus salmonis isolated from pyrethroid resistant strain (Ls_Viknal Cytb) |
| 6 | Oligonucleotide (forward) primer useful for detecting SNP T8605C |
| 7 | Oligonucleotide (reverse) primer useful for detecting SNP T8605C |
| 8 | Oligonucleotide (forward) primer useful for detecting SNP A9035G |
| 9 | Oligonucleotide (reverse) primer useful for detecting SNP A9035G |
| 10 | Oligonucleotide (forward) primer useful for detecting SNP C13957T |
| 11 | Oligonucleotide (reverse) primer useful for detecting SNP C13957T |
| 12 | Oligonucleotide (forward) primer useful for detecting SNP A14017G |
| 13 | Oligonucleotide (reverse) primer useful for detecting SNP A14017G |
| 14 | Oligonucleotide (forward) primer useful for detecting SNP C14065T |
| 15 | Oligonucleotide (reverse) primer useful for detecting SNP C14065T |
| 16 | Oligonucleotide probe useful for detecting SNP T8605C |
| 17 | Oligonucleotide probe useful for detecting SNP A9035G |
| 18 | Oligonucleotide probe useful for detecting SNP C13957T |
| 19 | Oligonucleotide probe useful for detecting SNP A14017G |
| 20 | Oligonucleotide probe useful for detecting SNP C14065T |

According to one embodiment of the present invention, a method is provided for detection of the pyrethroids deltamethrin (CAS No. 52918-63-5) and cis-cypermethrin (CAS No. 52315-07-8). It is well known that resistance against one type of pyrethroid in general provides the possibility of resistance also against all type of pyrethroids; see e.g. Sevatdal S, Fallang A, Ingebrigtsen K, Horsberg TE. 2005. Monooxygenase mediated pyrethroid detoxification in sea lice (Lepeophtheirus salmonis). Pest Manag Sci. 2005 Aug;61 (8):772-8, Du Y, Nomura Y, Satar G, Hu Z, Nauen R, He SY, Zhorov BS, Dong K. 2013. Molecular evidence for dual pyrethroid-receptor sites on a mosquito sodium channel. Proc Natl Acad Sci U S A. 2013 Jul 2. [Epub ahead of print], and Zhu F, Gujar H, Gordon JR, Haynes KF, Potter MF, Palli SR. 2013. Bed bugs evolved unique adaptive strategy to resist pyrethroid insecticides. Sci Rep. 2013;3:1456. doi: 10.1038/srep01456. The skilled person will thus acknowledge, based on the teaching of the present invention, that the method according to the invention may be used to determine resistance towards various types of pyrethroids in crustaceans, such as sea lice.

### Examples

### Example 1: Hybridization of sensitive and pyrethroid resistant sea lice

### Crossing experiment

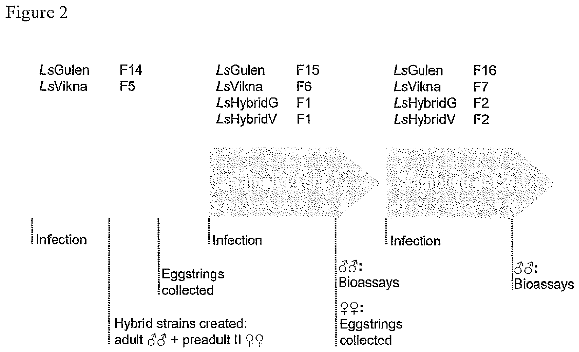

In order to investigate the inheritance and mechanism of pyrethroid resistance in *L*. *salmonis,* two strains (sensitive and resistant) and their reciprocal hybrids were used for a two-generation breeding experiment complimented with bioassays to measure tolerance (Fig. 2). The pyrethroid sensitive strain (hereon also referred to as *Ls*Gulen) was collected in June 2006 from newly slaughtered rainbow trout from Gulen in western Norway. The pyrethroid resistant strain (hereon also referred to as *Ls*Vikna) was collected in February 2009 from a salmon farm in Vikna, North Trøndelag, Norway. The resistant strain was collected on the basis of suspected reduced sensitivity to pyrethroids due to the fact that there had been reported a treatment failure on the farm from which it was collected. This was subsequently confirmed in bioassays with *Ls*Gulen as sensitive control.

Prior to the initiation of the present study, *Ls*Gulen and *Ls*Vikna had been cultivated in laboratory for 13 and 4 generations respectively under standard rearing conditions at the Institute of Marine Research (IMR), Norway (Hamre et al., 2009, Establishment and characterisation of salmon louse (Lepeophtheirus salmonis (Krøyer 1837)) laboratory strains. Parasitology International, 58, 451-460).

In order to hybridise the lice strains for the inheritance experiment, the parental strains were first synchronised and multiplied up in numbers by simultaneously infecting 20 Atlantic salmon with copepods. In total, 1000 copepods per strain were added. At 32 days post infection (dpi), all salmon were anaesthetised and approximately half of the lice were carefully removed using tweezers. At this stage, lice were predominantly preadult 2 females and adult males, with a few percent adult females and preadult 2 males. The lice removed from the salmon were sexed, and then used to create the reciprocal hybrid strains. This was achieved by combining 4-5 females from the resistant strain and 4-5 males from the sensitive strain (*Ls*HybridV), and 4-5 females from the sensitive strain and 4-5 males from the resistant strain (*Ls*HybridG). Only preadult 2 females were used. These combinations were each placed into 2 replicate tanks in order to make two hybrid strains (4 groups and 8 tanks at this stage). At 67-68 dpi, all lice were removed from the four strains. Egg strings were removed from adult females and incubated in containers containing a single strain each. The number of adult lice, and harvested egg strings varied, but all were incubated (Table 2).

**Table 2. Number of egg strings collected from each strain. Egg strings from replicate tanks were pooled. Most egg strings were part of a pair.**

| Strain | Generation | Egg strings (n) |
|---|---|---|
| *Ls*Gulen | F14 | 133 |
| *Ls*Vikna | F5 | 106 |
| *Ls*HybridG | P | 96 |
| *Ls*HybridV | P | 60 |

The subsequent generation (F2, Fig. 2) were both produced using the general procedure described above, although the hybrid strains were only crossed back to themselves to create multiple generation-hybrids. Collection of egg strings, which marked the establishment of a new generation, was done at 55 and 56 dpi respectively. Salmon that hosted the previous generation were re-used for reinfection with the next generation, but hosted only the same experimental strain.

### Bioassay test

Each strain, including both types of hybrids obtained in example 1 were quantified in their tolerance of deltamethrin at sampling points 1 and 2 representing the F1 and F2 generations for the hybrids (Fig. 1 and 2). Bioassays were performed according to standard guidelines (SEARCH Consortium, 2006, Sea lice resistance to chemotherapeutants: A handbook in resistance management. 2 ed.), with minor modifications. Only adult male lice were used for the bioassay, which after sampling (at 55 or 56 dpi) had been incubated overnight in running seawater (ca. 10 °C). The 30 min exposure to the prepared deltamethrin concentration was also carried out at ca. 10 °C, and higher concentrations than those described in the given protocol were included (Table S3). After exposure to deltamethrin, the lice were kept in running seawater (24 h at ca. 10 °C) until evaluation of the bioassays. At evaluation, lice were characterised as active, or inactive (moribund and dead lice pooled). EC₅₀ values were calculated using probit analysis (PoloPlus 2.0; LeOra Software, CA, USA). The results of two deltamethrin bioassay sets are shown in table 3a and 3b below.

**Table 3a. Deltamethrin bioassays performed on sampling set 1**

| | | | |
|---|---|---|---|
| Lice: | LsHybridG | | |
| Generation: | F1 | | |
| Fish tank: | 01 | | |

| Dose (ppb) | Active (n) | Inactive (n) | % Inactive |
|---|---|---|---|
| 0 | 17 | 0 | 0 |
| 0.1 | 16 | 0 | 0 |
| 0.3 | 32 | 9 | 21 |
| 1 | 0 | 35 | 100 |
| 3 | 0 | 28 | 100 |
| 6 | 0 | 18 | 100 |
| 12 | 0 | 27 | 100 |
| | | | |
| Lice: | LsHybridV | | |
| Generation: | F1 | | |
| Fish tank: | 02 | | |
| 0 | 16 | 0 | 0 |
| 0.1 | 17 | 0 | 0 |
| 0.3 | 44 | 0 | 0 |
| 1 | 56 | 3 | 5 |
| 3 | 33 | 5 | 13 |
| 6 | 17 | 1 | 5 |
| 12 | 15 | 7 | 31 |
| | | | |
| Lice: | LsGulen | | |
| Generation: | F15 | | |
| Fish tank: | 04 | | |

| Dose (ppb) | Active (n) | Inactive (n) | % Inactive |
|---|---|---|---|
| 0 | 9 | 0 | 0 |
| 0.1 | 40 | 0 | 0 |
| 0.3 | 29 | 27 | 48 |
| 1 | 0 | 37 | 100 |
| 3 | 0 | 20 | 100 |
| 6 | 0 | 14 | 100 |
| 12 | 0 | 9 | 100 |
| | | | |
| Lice: | LsVikna | | |
| Generation: | F6 | | |
| Fish tank: | 05 | | |

| Dose (ppb) | Active (n) | Inactive (n) | % Inactive |
|---|---|---|---|
| 0 | 12 | 0 | 0 |
| 0.1 | 16 | 0 | 0 |
| 0.3 | 9 | 0 | 0 |
| 1 | 21 | 3 | 12 |
| 3 | 29 | 11 | 27 |
| 6 | 18 | 10 | 35 |
| 12 | 7 | 5 | 41 |
| | | | |
| Lice: | LsHybridV | | |
| Generation: | F1 | | |
| Fish tank: | 06 | | |

| Dose (ppb) | Active (n) | Inactive (n) | % Inactive |
|---|---|---|---|
| 0 | 26 | 0 | 0 |
| 0.1 | 17 | 0 | 0 |
| 0.3 | 45 | 0 | 0 |
| 1 | 39 | 3 | 7 |
| 3 | 36 | 1 | 2 |
| 6 | 19 | 2 | 9 |
| 12 | 19 | 9 | 32 |
| Lice: | LsHybridG | | |
| Generation: | F1 | | |
| Fish tank: | 07 | | |

| Dose (ppb) | Active (n) | Inactive (n) | % Inactive |
|---|---|---|---|
| 0 | 14 | 0 | 0 |
| 0.1 | 18 | 4 | 18 |
| 0.3 | 21 | 10 | 32 |
| 1 | 0 | 38 | 100 |
| 3 | 0 | 46 | 100 |
| 6 | 0 | 21 | 100 |
| 12 | 0 | 20 | 100 |
| | | | |
| Lice: | LsVikna | | |
| Generation: | F6 | | |
| Fish tank: | 09 | | |

| Dose (ppb) | Active (n) | Inactive (n) | % Inactive |
|---|---|---|---|
| 0 | 16 | 0 | 0 |
| 0.1 | 21 | 1 | 4 |
| 0.3 | 15 | 0 | 0 |
| 1 | 26 | 3 | 10 |
| 3 | 17 | 4 | 19 |
| 6 | 23 | 7 | 23 |
| 12 | 5 | 2 | 28 |
| | | | |
| Lice: | LsGulen | | |
| Generation: | F15 | | |
| Fish tank: | 10 | | |

| Dose (ppb) | Active (n) | Inactive (n) | % Inactive |
|---|---|---|---|
| 0 | 13 | 0 | 0 |
| 0.1 | 26 | 0 | 0 |
| 0.3 | 10 | 10 | 50 |
| 1 | 0 | 41 | 100 |
| 3 | 0 | 9 | 100 |
| 6 | 0 | 17 | 100 |
| 12 | 0 | 22 | 100 |

**Table 3b. Deltamethrin bioassays performed on sampling set 2 .**

| | | | |
|---|---|---|---|
| Lice: | LsGulen | | |
| Generation: | F16 | | |
| Fish tank: | 03 | | |

| Dose (ppb) | Active (n) | Inactive (n) | % Inactive |
|---|---|---|---|
| 0 | 19 | 0 | 0 |
| 0.1 | 16 | 1 | 5 |
| 0.3 | 28 | 26 | 48 |
| 0.5 | 7 | 43 | 86 |
| 1 | 0 | 18 | 100 |
| 3 | 0 | 23 | 100 |
| 6 | 0 | 7 | 100 |
| | | | |
| Lice: | LsGulen | | |
| Generation: | F16 | | |
| Fish tank: | 04 | | |

| Dose (ppb) | Active (n) | Inactive (n) | % Inactive |
|---|---|---|---|
| 0 | 24 | 0 | 0 |
| 0.1 | 23 | 2 | 8 |
| 0.3 | 16 | 20 | 55 |
| 0.5 | 1 | 20 | 95 |
| 1 | 0 | 16 | 100 |
| 3 | 0 | 15 | 100 |
| 6 | 0 | 6 | 100 |
| 12 | 0 | 3 | 100 |
| | | | |
| Lice: | LsHybridV | | |
| Generation: | F2 | | |
| Fish tank: | 06 | | |

| Dose (ppb) | Active (n) | Inactive (n) | % Inactive |
|---|---|---|---|
| 0 | 19 | 0 | 0 |
| 0.1 | 13 | 0 | 0 |
| 0.3 | 14 | 0 | 0 |
| 0.5 | 15 | 0 | 0 |
| 1 | 32 | 0 | 0 |
| 3 | 25 | 5 | 16 |
| 6 | 22 | 1 | 14 |
| 12 | 11 | 3 | 21 |
| 20 | 6 | 4 | 40 |
| | | | |
| Lice: | LsHybridG | | |
| Generation: | F2 | | |
| Fish tank: | 07 | | |

| Dose (ppb) | Active (n) | Inactive (n) | % Inactive |
|---|---|---|---|
| 0 | 22 | 0 | 0 |
| 0.1 | 17 | 0 | 0 |
| 0.3 | 14 | 14 | 50 |
| 0.5 | 1 | 6 | 85 |
| 1 | 1 | 10 | 90 |
| 3 | 0 | 18 | 100 |
| 6 | 0 | 32 | 100 |
| 12 | 0 | 27 | 100 |
| 20 | 0 | 32 | 100 |
| | | | |
| Lice: | LsVikna | | |
| Generation: | F7 | | |
| Fish tank: | 08 | | |

| Dose (ppb) | Active (n) | Inactive (n) | % Inactive |
|---|---|---|---|
| 0 | 35 | 0 | 0 |
| 0.1 | 9 | 0 | 0 |
| 0.3 | 27 | 0 | 0 |
| 0.5 | 6 | 0 | 0 |
| 1 | 12 | 0 | 0 |
| 3 | 14 | 1 | 6 |
| 6 | 22 | 5 | 18 |
| 12 | 6 | 0 | 0 |
| 20 | 17 | 11 | 39 |
| | | | |
| Lice: | LsHybridV | | |
| Generation: | F2 | | |
| Fish tank: | 09 | | |

| Dose (ppb) | Active (n) | Inactive (n) | % Inactive |
|---|---|---|---|
| 0 | 16 | 0 | 0 |
| 0.1 | 12 | 2 | 14 |
| 0.3 | 11 | 0 | 0 |
| 0.5 | 28 | 1 | 3 |
| 1 | 17 | 1 | 5 |
| 3 | 17 | 0 | 0 |
| 6 | 25 | 6 | 19 |
| 12 | 11 | 1 | 8 |
| 20 | 14 | 17 | 54 |
| Lice: | | | |
| | LsHybridG | | |
| Generation: | F2 | | |
| Fish tank: | 10 | | |

| Dose (ppb) | Active (n) | Inactive (n) | % Inactive |
|---|---|---|---|
| 0 | 10 | 0 | 0 |
| 0.1 | 19 | 1 | 5 |
| 0.3 | 25 | 19 | 43 |
| 0.5 | 6 | 16 | 72 |
| 1 | 0 | 10 | 100 |
| 3 | 0 | 23 | 100 |
| 6 | 0 | 19 | 100 |
| 12 | 0 | 25 | 100 |
| 20 | 0 | 10 | 100 |

The four experimental strains were successfully cultured through the two generations and exposed to two separate bioassays. At the control and lowest concentration (0.1 ppb), no differences in lice activity was observed between the four strains. This was the case for both sampling 1 and 2. However, at concentrations between 0.3 and 1 ppb, clear differences were observed between the strains (Fig. 2). Above 1 ppb, only lice from the resistant strain and its maternal hybrid strain survived the bioassay, while the sensitive strain and its associated maternal hybrid strain displayed 100% mortality. No differences between the hybrid strain and its maternal founder strain were observed for either strain, demonstrating that the resistance followed a maternal pattern of inheritance.

### Exemple 2: Identification of SNPs associated with pyrethroid resistance

The presented outcome of crossing resistant and sensitive lice (example 1) showed that the mitochondria genome encodes the resistance. The L. salmonis mtDNA was characterized by Tjensvoll et al (2005) and encodes 13 proteins, two rRNAs and 22 tRNA genes.

The first approach to identify genetic differences between the LsSensitive (LsGulen) and Ls Resistent (LsVikna) was by sequencing a 1000 bp fragment from *cytB* from 6 *L*. *salmonis* specimens (3 sensitive and 3 resistant). The sequencing revealed 3 unique SNPs in the *cytB* from the LsResistent strain. We sequenced the *cytB* from 10 more individual from the LsResistent strain, which revealed that all except 1 individual had the same unique SNP (C14065T) in the *cytB.* This single atypical LsVikna individual was sampled from the control group during the bioassay (i.e. LsVikna incubated without deltamethrin). Tjensvoll et al (2006) sequenced *cytB, A6. col* and *16S* from 180 *L. salmonis* specimens from six locations in the North Atlantic that were collected in 2000 or 2002. We aligned our cytB (from LsSensitive and LsResistent) sequences to these 180 cytB sequences from Tjensvoll et al (2006). None of the 180 cytB sequences from historic samples had the C14065T SNP. To further validate if the C14065T SNP is unique in resistant lice we obtained samples from 9 different lice strains, including several field strains with reduced sensitivity towards deltamethrin or cis-cypermethrin.

The presented outcome of crossing resistant and sensitive lice showed that the mitochondria genome encodes the resistance. Since *L. salmonis* is ZZ (male)/Z0 (female) the maternally encoded resistance properties must be in the mtDNA. The *L. salmonis* mtDNA was characterized by Tjensvoll et al (2005) and encodes 13 proteins, two rRNAs and 22 tRNA genes.

### Genetic variation within the mtDNA

Tjensvoll et al (2006), *supra,* identified 158 haplotypes for cytb and 164 haplotypes for COI. In 19 *L. salmonis* individuals with know deltamethrin resistance only 2 haplotypes for *COI* were identified. The cytB was sequenced from 29 individuals with known deltamethrin resistance and only 1 haplotype was identified. Among 19 sensitive specimens 9 different haplotypes for cytB was present (Table 4).

**Table 4. Number of haplotypes identified for each of CytB and Col in sensitive sea lice and resistant sea lice.**

| | **CytB** | **Col** |
|---|---|---|
| **Tjensvoll et al** | 158 (180) | 164 (180) |
| **Sensitive lice** | 9 (19) | 11 (19) |
| **Resistant lice** | 1 (29) | 2 (19) |

### Example 3: Testing for PRLS

Based on the identification of SNPs responsible for pyrethroid resistance in *L*. *salmonis,* we developed several sensitive Real-Time PCR (TaqMan) 5'-nuclease assays for single nucleotide polymorphism (SNP) detection (Real-Time PCR SNP-assay) (Table 1), and successfully applied these to differentiate between pyrethroid resistant and sensitive sea lice. Fluorogenic PCR probes, chemically modified with a minor groove binding agent to increase duplex stability, were used in single and multiplex probe closed tube formats. The probes were tested in a commercially available thermocycling fluorimeter (Applied Biosystems 7500 Real-Time PCR System) at PatoGen Analyse AS laboratory in Alesund. All assays were used qualitatively to determine the relative amount of the SNP- in individual samples, and samples from different life stages of sea lice were use. Comparison of results obtained using sea lice samples with different Real-Time PCR SNP-assays detecting different SNPs specific for pyrethroid resistant sea lice showed no discrepancies from results obtained by genome sequencing. The reported SNPs and the relevant Real-Time PCR SNP-assays are ideal for the differentiating between pyrethroid resistant and sensitive sea lice in the aquaculture industry.

### Sampling of sea lice

Sea lice samples were collected from sea lice with known sensitivity-status to pyrethroids, cultivated in The Sea Lice Research Centre laboratories at the University of Bergen. The sea lice had been tested with respect to sensitivity to pyrethroids by bioassays as previously described in example 1 above. Also, the genome sequences for the relevant genes were known from previous genomic sequencing performed as described in example 1 above.

In addition, sea lice were sampled from fish farms on the west coast of Norway. Sea lice were collected using forceps, and approximately 10-50 lice per site were conserved in 70% ethanol and kept at 4°C. Samples were sent refrigerated to PatoGen by express mail carrier.

### Nucleic acid purification

In PatoGens laboratory, RNA and/or DNA were extracted from samples by methods well known to the skilled person. In short, tissue samples were transferred to Micro Collection Tubes and lysed and homogenized using QIAzol Lysis Reagent, steel beads and vigorous shaking using a TissueLyser system, followed by nucleic acid extraction using an RNAeasy kit (Qiagen) or DNAeasy kit (Qiagen), all according to the manufacturer's instructions, and by methods well known to the skilled person. Chloroform were added to the samples and shaken vigorously. After resting and centrifuging, the relevant liquid phase were collected for further extraction of either RNA or DNA by vacuum technology using a Qiagen robot system, all according to the manufacturer's instructions, and by methods well known to the skilled person. Finally, nucleic acids were eluted in 25 ml of elution buffer and used for PCR by methods well known to the skilled person.

### SNP-detection by Real-Time PCR

The primers and probes listed in table 5 are TaqMan® MGB Probe SNP Genotyping Assays using TaqMan® 5' nuclease assay chemistry for amplifying and detecting specific SNP alleles in purified genomic DNA or RNA. In this study, primers and probes SNP-14017, SNP-14065, & SNP-9035 listed in table 1 were ordered from Life Technologies Corporation. The primers and probes listed in table 1 for SNP-8605 and SNP-13957 serves as examples of assays for these SNPs, but were not included in this study. One-step amplification (45 cycles) was performed on an Applied Biosystems 7500 Real-Time PCR System performed at PatoGen Analyse AS laboratory in Alesund, all according to the manufacturer's instructions, and by methods well known to the skilled person. All assays were tested on both RNA and DNA, and used qualitatively to determine the relative amount of the relevant SNPs in individual samples.

**Table 5: Primers and probes**

| **Assay ID** | **Position AY6298 57** | **Forward primer** | **Revers primer** | **Probe with IUPAC nucleotide code** | **Probe** | **Sensitive** | **Resistant** |
|---|---|---|---|---|---|---|---|
| SNP-8605 | T8605C | | | CCTCTT**Y**GAGTTTATT (SEQ ID No. 16) | | T | C |
| SNP-9035 | A9035G | | | TTCCAGGGTTTGG**R**TTA (SEQ ID No. 17) | | A | G |
| SNP-13957 | C13957T | | | | | C | T |
| SNP-14017 | A14017 G | | | CAGCAAAAATGG**R**AA (SEQ ID No. 19) | | A | G |
| SNP-14065 | C14065T | | | CCCCCC**Y**AACTTAT (SEQ ID No. 20) | | C | T |

**Table 5: Primers and probes.** Listing of primers and probes for each SNP identified. Probes are both listed with IUPAC nucleotide codes for SNPs, and with the alternative nucleotides for each position as indicated. IUPAC codes used are in accordance with Nomenclature for Incompletely Specified Bases in Nucleic Acid Sequences, of which the following are relevant here: Y = C or T, R = A or G (Nomenclature Committee of the International Union of Biochemistry (NC-IUB) (1984) htt://www.chem.qmul.ac.uk/iubmb/misc/naseq.html).

### Results

The results from Real-Time PCR-assays are interpreted by looking at the deviation in Ct-values between the probes detecting the two variants of the SNP. For the Real-Time PCR-assay towards SNP-14017 performed using RNA or DNA, sensitive lice has a deviation lower than -2, and resistant sea lice has a deviation higher than -2. For the Real-Time PCR-assays towards SNP-14065 & SNP-9035 performed using RNA or DNA, sensitive lice has a deviation lower than zero, and resistant sea lice has a deviation higher than zero. In addition, there is a quantitative aspect where the highly resistant strains have a higher deviation value than moderately resistant sea lice. Most likely, genetically resistant strains that have not been exposed to pyrethroids for some time has a lower deviation value than genetically resistant strains that been repeatedly and newly exposed to pyrethroids.

The results from the Real-Time PCR SNP-analyses using assays directed towards SNP-14065 & SNP-9035 (according to Table 5) using RNA or DNA from sea lice samples with known sensitivity to pyrethroids showed good correlation with results obtained by genome sequencing, and correlated with the known resistance status in sea lice populations (Table 6 & 7). The Real-Time PCR SNP-assay directed towards SNP-14017 (according to Table 5) gave the same results using DNA or RNA, and correlated with the results from SNP-14065 & SNP-9035 for all but one sea lice sample (Table 6).

Also, analyses performed on a higher number of sampled field strains showed good correlation with the known resistance status and as shown in Table 8. Here, analyses were performed using SNP-14065, and RNA as template.

All tested Real-Time PCR-SNP-assays show very promising results, and we believe that these SNPs can be used separately and/or in combination with other SNPs to serve as a tool in the practical differentiation between pyrethroid- sensitive and resistant sea lice.

Thus the reported SNPs and the relevant Real-Time PCR SNP-assays represent an ideal tool for differentiating between pyrethroid resistant and sensitive sea lice in the aquaculture industry. Also, the prevalence of sensitive versus resistant sea lice in a population, and the deviation value for individual lice or average deviation values for populations, can be used to predict the best possible outcome of a treatment using pyrethroids in the population. Also, the technique can become an important tool for optimizing sea lice treatments using pyrethroids, and to monitor the resistant status of populations of sea lice before treatment.

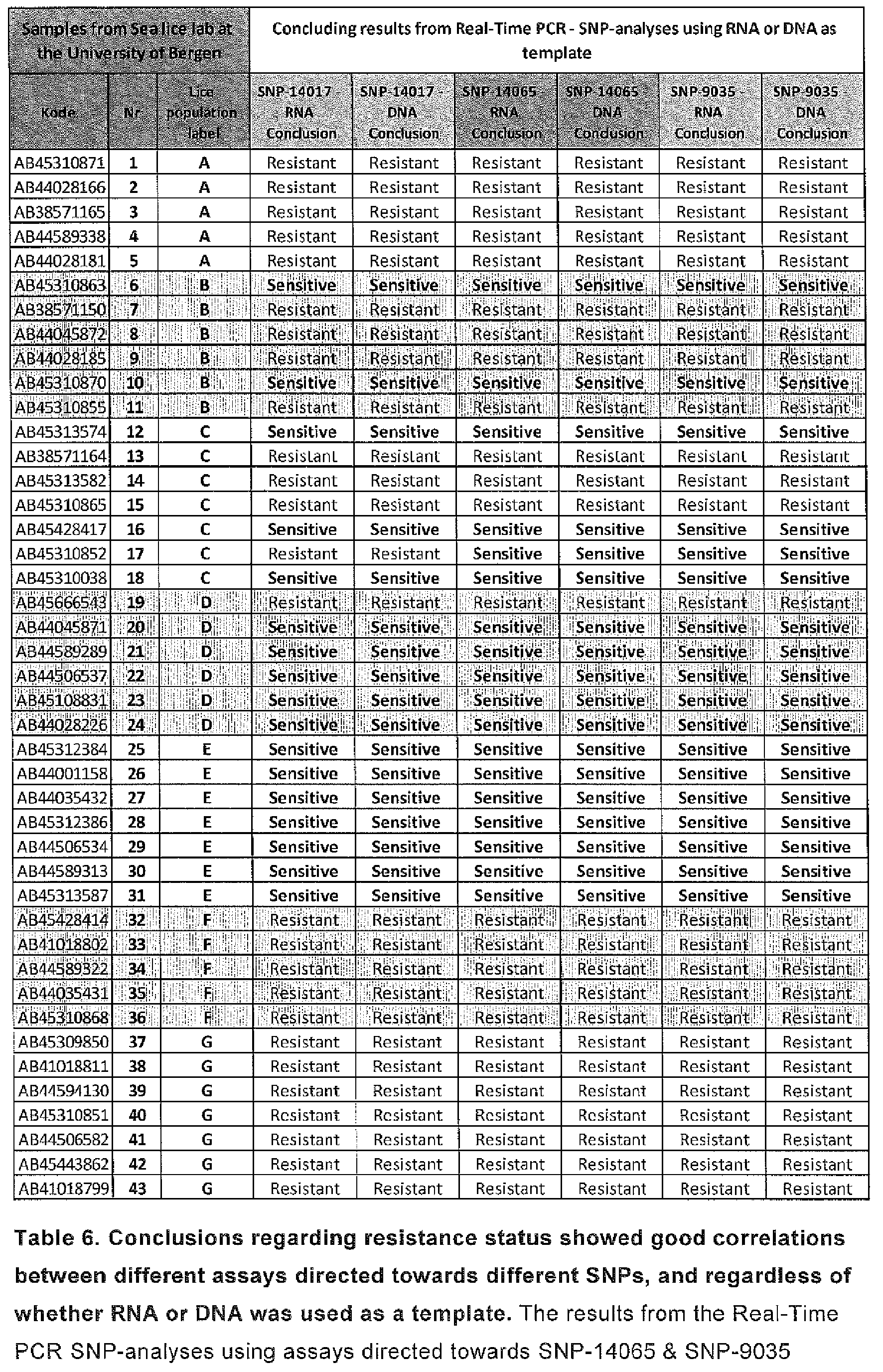

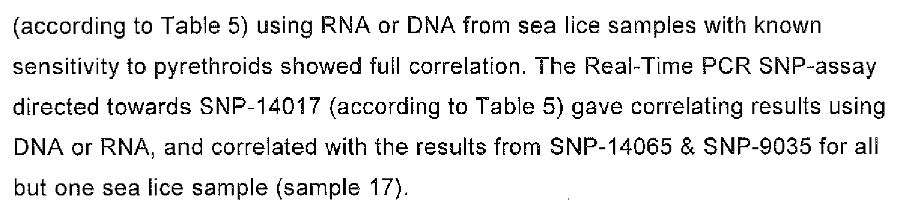

**Table 7. SNP-analyses correlate well with known resistance status. SNP-analyses using SNP14065 correlates with known resistance status in sea lice (L. salmonis) from the sea lice lab at the University of Bergen (based on bioassays and field observations), and with genotyping performed by the University of Bergen. Bioassays, genotyping and SNP-analyses are all based on different lice from the same populations, and thus the results cannot be correlated on individual sea lice level. Also, since the number of samples is very low, the slight differences with respect to prevalence of resistant genotype observed between sequencing and Real-Time PCR SNP-14065-analysis must be attributed to the low number of samples tested, and this is especially the case for lice population D. Still, the conclusions with respect to population resistance level correlates well. Also, populations A, B and C represent surviving sea lice from the same original population, but A, B and C are surviving lice that has been exposed to different concentrations of pyrethroid in a bioassay. The results show that pyrethroid treatment selects for more resistant sea lice, and this is reflected in both the prevalence of genetically resistant lice, and in the total average deviation in Ct-values between the probes detecting the two variants of the SNP.**

| **Samples from Sea lice lab at the University of Bergen** | | | **Results from genotyping CytB - 14065** | | | | **Results Real-Time PCR - SNP-14065** | | | | | | | | |
|---|---|---|---|---|---|---|---|---|---|---|---|---|---|---|---|
| **Lice population label** | **Isolat description/bio-assay conclusions** | | **# tested** | **# Resistant** | **# Sensitive** | **Prevalence of resistant** | **# tested** | **# Resistant** | **# Sensitive** | **% prevalence sensitive** | **% prevalence resistant** | **Average deviation sensitive** | **Average deviation resistant** | **Total average deviation** | **Conclusion resistance status** |
| A | Surviving lice from bioassay | Highest dose | 5 | 5 | 0 | 100 | 5 | 5 | 0 | 0 | 100 | - | 12,9 | 12,9 | Resistant |
| B | Surviving lice from bioassay | Second highest dose | 2 | 1 | 1 | 50 | 6 | 4 | 2 | 33 | 67 | -1,5 | 13,7 | 8,63 | Resistant |
| C | Surviving lice from bioassay | Control | 6 | 2 | 4 | 33 | 7 | 3 | 4 | 57 | 43 | -1,6 | 12,6 | 4,53 | Reduced sensitivity |
| D | HF | Slightly reduced sensitivity | 3 | 2 | 1 | 67 | 6 | 1 | 5 | 83 | 17 | -1,3 | 12,9 | 1,03 | Slightly reduced sensitivity |
| E | LG | Sensitive | 3 | 0 | 3 | 0 | 7 | 0 | 7 | 100 | 0 | -1,4 | | -1,36 | Sensitive |
| F | LF | Resistant | 13 | 12 | 1 | 92 | 5 | 5 | 0 | 0 | 100 | - | 11,8 | 11,84 | Resistant |
| G | LB | Multi-resistant | 10 | 10 | 0 | 100 | 7 | 7 | 0 | 0 | 100 | - | 10,3 | 10,34 | Resistant |

**Table 8. SNP-analyses correlate well with known resistance status in field strains. Field strains of sea lice (L. salmonis) were characterized with respect to sensitivity to pyrethroids at the University of Bergen sea lice lab, and with the exception of strain P, all strains have been cultivated for one or more generations in the lab (up to 34 generations) before sampling. The conclusions from Real-Time PCR analyses towards SNP-14065 correlates very well with the known resistance characteristics from bioassays and field observations.**

| **Sample from Sea lice lab at the University of Bergen** | | **Results Real-Time PCR - SNP-14065** | | | | | | | | |
|---|---|---|---|---|---|---|---|---|---|---|
| **Lice population label/ bio-assay conclusions** | | **n** | **# Resistant** | **# Sensitive** | **% prevalence sensitive** | **% prevalence resistant** | **Average deviation sensitive** | **Average deviation resistant** | **Total average deviation** | **Conclusion resistance status** |
| H | Sensitive | 30 | 0 | 30 | 100 | 0 | -1,7 | - | -1,7 | Sensitive |
| I | Resistant | 30 | 30 | 0 | 0 | 100 | - | 5,5 | 5,5 | Reduced sensitivity |
| J | Resistant | 30 | 30 | 0 | 0 | 100 | - | 5,1 | 5,1 | Reduced sensitivity |
| K | Sensitive | 30 | 0 | 30 | 100 | 0 | -1,3 | - | -1,3 | Sensitive |
| L | Sensitive | 30 | 0 | 30 | 100 | 0 | -1,7 | - | -1,7 | Sensitive |
| M | Sensitive | 30 | 0 | 30 | 100 | 0 | -1,4 | - | -1,4 | Sensitive |
| N | Sensitive | 7 | 0 | 7 | 100 | 0 | -1,4 | - | -1,4 | Sensitive |
| O | Sensitive | 28 | 0 | 28 | 100 | 0 | -1,3 | - | -1,3 | Sensitive |
| P | Slightly reduced sensitivity | 30 | 1 | 29 | 97 | 3 | -0,7 | 10,7 | -0,3 | Sensitive (Slightly reduced sensitivity) |

### FUTURE EXAMPLES

In addition to the specific examples above related to *L. salmonis*, and in order to confirm the applicability of the observations for *L. salmonis* to other arthropods, in particular crustaceans, in particular copepods, the inventors plan to execute the following experimental protocol:

### Crossing experiment Caligus elongatus

In order to investigate the inheritance and mechanism of pyrethroid resistance in *Caligus elongatus,* two strains (sensitive and resistant) and their reciprocal hybrids will be used for a two-generation breeding experiment complimented with bioassays to measure tolerance. A pyrethroid sensitive strain and a pyrethroid resistant strain will be selected on the basis of results from bioassays.

In order to hybridise the lice strains for the inheritance experiment, the parental strains will first be synchronised and multiplied up in numbers by simultaneously infecting 20 Atlantic salmon with copepodids. At the stage when lice are predominantly preadult 2 females and adult males, the lice will removed from the salmon, sexed, and then used to create the reciprocal hybrid strains. This will be achieved by combining 4-5 females from the resistant strain and 4-5 males from the sensitive strain, and 4-5 females from the sensitive strain and 4-5 males from the resistant strain. Only preadult 2 females will be used. These combinations will each be placed into 2 replicate tanks in order to make two hybrid strains (4 groups and 8 tanks at this stage). When egg strings develop in these hybrid strains, all lice will be removed from the four strains. Egg strings will be removed from adult females and incubated in containers containing a single strain each.

The subsequent generation (F2) will be produced using the general procedure described above, although the hybrid strains will only be crossed back to themselves to create multiple generation-hybrids. Collection of egg strings will mark the establishment of a new generation. Salmon that hosted the previous generation will be re-used for reinfection with the next generation, but will only host the same experimental strain.

### Bioassay test

Each strain, including both types of hybrids obtained in this example will be quantified in their tolerance of deltamethrin at sampling points 1 and 2 representing the F1 and F2 generations for the hybrids. Bioassays will be performed according to standard guidelines (SEARCH Consortium, 2006, Sea lice resistance to chemotherapeutants: A handbook in resistance management. 2 ed.), with minor modifications. Only adult male lice will be used for the bioassay, which after sampling will be incubated overnight in running seawater (ca. 10 °C). The 30 min exposure to the prepared deltamethrin concentration will also be carried out at ca. 10 °C, and higher concentrations than those described in the given protocol will be included. After exposure to deltamethrin, the lice will be kept in running seawater (24 h at ca. 10 °C) until evaluation of the bioassays. At evaluation, lice will be characterised as active, or inactive (moribund and dead lice pooled). EC₅₀ values will be calculated using probit analysis (PoloPlus 2.0; LeOra Software, CA, USA).

The expected results from this experiment is that only lice from the resistant strain and its maternal hybrid strain will show resistance towards the pyrethroid treatment, and thus survive the bioassay. The sensitive strain and its associated maternal hybrid strain will displayed close to 100% mortality. No differences between the hybrid strain and its maternal founder strain will be observed for either strain, demonstrating that the resistance follow a maternal pattern of inheritance.

The presented outcome of crossing resistant and sensitive lice will show that the mitochondria genome encodes the resistance in this species. We will then amplify, sequence, and compare complete mitochondrial genomes of susceptible and resistant strains of *Caligus elongatus* strains to identify the specific SNPs associated to this resistance, but the above described experiment will provide enough information to conclude that the mitochondria is involved in the regulation of resistance towards pyrethroids in *Caligus elongatus* as described for the *L*. *salmonis.*

### Crossing experiment Caligus rogercresseyi

In order to investigate the inheritance and mechanism of pyrethroid resistance in *Caligus rogercresseyi,* two strains (sensitive and resistant) and their reciprocal hybrids will be used for a two-generation breeding experiment complimented with bioassays to measure tolerance. A pyrethroid sensitive strain and a pyrethroid resistant strain will be selected on the basis of results from bioassays.

In order to hybridise the lice strains for the inheritance experiment, the parental strains will first be synchronised and multiplied up in numbers by simultaneously infecting 20 Atlantic salmon with copepodids. At the stage when lice are predominantly preadult 2 females and adult males, the lice will removed from the salmon, sexed, and then used to create the reciprocal hybrid strains. This will be achieved by combining 4-5 females from the resistant strain and 4-5 males from the sensitive strain, and 4-5 females from the sensitive strain and 4-5 males from the resistant strain. Only preadult 2 females will be used. These combinations will each be placed into 2 replicate tanks in order to make two hybrid strains (4 groups and 8 tanks at this stage). When egg strings develop in these hybrid strains, all lice will be removed from the four strains. Egg strings will be removed from adult females and incubated in containers containing a single strain each.

The subsequent generation (F2) will be produced using the general procedure described above, although the hybrid strains will only be crossed back to themselves to create multiple generation-hybrids. Collection of egg strings will mark the establishment of a new generation. Salmon that hosted the previous generation will be re-used for reinfection with the next generation, but will only host the same experimental strain.

### Bioassay test

Each strain, including both types of hybrids obtained in this example will be quantified in their tolerance of deltamethrin at sampling points 1 and 2 representing the F1 and F2 generations for the hybrids. Bioassays will be performed according to standard guidelines (SEARCH Consortium, 2006, Sea lice resistance to chemotherapeutants: A handbook in resistance management. 2 ed.), with minor modifications. Only adult male lice will be used for the bioassay, which after sampling will be incubated overnight in running seawater (ca. 10 °C). The 30 min exposure to the prepared deltamethrin concentration will also be carried out at ca. 10 °C, and higher concentrations than those described in the given protocol will be included. After exposure to deltamethrin, the lice will be kept in running seawater (24 h at ca. 10 °C) until evaluation of the bioassays. At evaluation, lice will be characterised as active, or inactive (moribund and dead lice pooled). EC₅₀ values will be calculated using probit analysis (PoloPlus 2.0; LeOra Software, CA, USA).

The expected results from this experiment is that only lice from the resistant strain and its maternal hybrid strain will show resistance towards the pyrethroid treatment, and thus survive the bioassay. The sensitive strain and its associated maternal hybrid strain will displayed close to 100% mortality. No differences between the hybrid strain and its maternal founder strain will be observed for either strain, demonstrating that the resistance follow a maternal pattern of inheritance.

The presented outcome of crossing resistant and sensitive lice will show that the mitochondria genome encodes the resistance in this species. We will then amplify, sequence, and compare complete mitochondrial genomes of susceptible and resistant strains of *Caligus rogercresseyi* strains to identify the specific SNPs associated to this resistance, but the above described experiment will provide enough information to conclude that the mitochondria is involved in the regulation of resistance towards pyrethroids in *Caligus rogercresseyi* as described for the *L*. *salmonis.*

Other experiments may be performed on other arthropods, in particular crustaceans, in particular copepods following similar experimental protocols as described herein, with expected similar results.

## Claims

1. An in vitro method for detection of pyrethroid resistance in a sea lice, comprising the steps of determining that the sea lice is a member of a species that exhibits inheritance of pyrethroid resistance from female members of the species, identifying a polymorphic site corresponding to a single nucleotide polymorphism (SNP) associated with pyrethroid resistance in the mitochondrial DNA of the species, and detecting the nucleotide at the polymorphic site in the organism to be analyzed.

2. The method according to claim 1, wherein the single nucleotide polymorphism (SNP) is present in a coding region of a gene encoding cytochrome B or in a coding region of a gene encoding the_cytochrome oxidase subunit I (COI).

3. The method according to claim 1, wherein the single nucleotide polymorphism (SNP) is present in a regulatory non-coding part of the mitochondrial DNA.

4. The method according to one of the preceding claims, wherein the sea lice is, L. salmonis, Caligus elongatus or Caligus rogercresseyi.

5. The method according to one of the preceding claims, wherein the sea lice is a member of a species having ZZ-ZO type sex determination.

6. The method according to one of the preceding claims, wherein the sea lice is a Siphonostomatoida.

7. The method according to one of the preceding claims, further comprising
a. collecting a sample of the organism from its natural environment;
b. isolating mitochondrial genomic material from the collected organism;
c. identifying the nucleotide at the polymorphic site of the isolated genomic material;
d. comparing the nucleotide polymorphic site of the isolated mitochondrial genomic material with a standard mitochondrial nucleic acid sequence from a member of the species known to be susceptible to pyrethroids, wherein the collected organism is determined to be resistant to pyrethroids if the nucleotide at the polymorphic site differs from the nucleotide at the polymorphic site of the standard.

8. The method according to claim 7, wherein step c) comprises nucleic acid amplification carried out by polymerase chain reaction.

9. The method according to claim 7, wherein step c) comprises contacting mitochondrial DNA sequence of the collected organism with a detection reagent, and determining which nucleotide is present at the polymorphic site.

10. A method according to claim 7, wherein step c) is performed using SNP specific probe hybridization, SNP specific primer extension, SPN specific amplification, sequencing, 5' nuclease digestion, molecular beacon assay, oligonucleotide ligation assay, size analysis, single-stranded conformation polymorphism analysis, or denaturing gradient gel electrophoresis.

11. A method for identifying a single nucleotide polymorphism indicative of pyrethroid resistance in a sea lice, comprising the steps of:
i.determining whether the species to which the sea lice belongs exhibits incidents of pyrethroid resistance,
ii.determining whether said pyrethroid resistance is inherited within the species from the female members to their progeny,
iii.in the event said pyrethroid resistance is inherited within the species from the female members to their progeny, then isolating mitochondrial DNA from a member of the species known to be resistant to pyrethroids and comparing said isolated DNA with the mitochondrial DNA of members of the species known to be susceptible to pyrethroids,
iv.identifying one or more single nucleotide polymorphisms in the mitochondrial DNA of the resistant member of the species,
v.performing a control to determine whether said single nucleotide polymorphisms are indicative of pyrethroid resistance.

12. The method according to claim 11, wherein the single nucleotide polymorphism (SNP) is present in a coding region of a gene encoding cytochrome B or in a coding region of a gene encoding the cytochrome oxidase subunit I (COI).

13. The method according to claim 11, wherein the single nucleotide polymorphism (SNP) is present in a regulatory non-coding part of the mitochondrial DNA.

14. The method according to one of claims 11-13, wherein the sea lice is, L. salmonis, Caligus elongatus or Caligus rogercresseyi.

15. The method according to one of claims 11-14, wherein the pest is a member of a species having ZZ-ZO type sex determination.

## Patentansprüche

1. In vitro-Verfahren zur Detektion von Pyrethroid-Resistenz bei Seeläusen, die Schritte umfassend, zu bestimmen, dass die Seeläuse Mitglieder einer Spezies sind, die ein Pyrethroid-Resistenz-Erbgut von weiblichen Mitgliedern der Spezies aufweist, eine polymorphe Stelle, die einem Einzelnukleotidpolymorphismus (SNP - single nucleotide polymorphism) entspricht, der mit Pyrethroid-Resistenz zusammenhängt, in der mitochondrialen DNA der Spezies zu identifizieren, und das Nukleotid an der polymorphen Stelle im zu analysierenden Organismus nachzuweisen.

2. Verfahren nach Anspruch 1, wobei der Einzelnukleotidpolymorphismus (SNP) in einer kodierenden Region eines Gens, das Cytochrom B kodiert, oder in einer kodierenden Region eines Gens vorhanden ist, das die Cytochromoxidase-Untereinheit I (COI) kodiert.

3. Verfahren nach Anspruch 1, wobei der Einzelnukleotidpolymorphismus (SNP) in einem regulatorischen, nicht kodierenden Teil der mitochondrialen DNA vorhanden ist.

4. Verfahren nach einem der vorhergehenden Ansprüche, wobei es sich bei den Seeläusen um L. salmonis, Caligus elongatus oder Caligus rogercresseyi handelt.

5. Verfahren nach einem der vorhergehenden Ansprüche, wobei es sich bei den Seeläusen um Mitglieder einer Spezies mit einer Geschlechtsbestimmung des Typs ZZ-ZO handelt.

6. Verfahren nach einem der vorhergehenden Ansprüche, wobei es sich bei den Seeläusen um Siphonostomatoida handelt.

7. Verfahren nach einem der vorhergehenden Ansprüche, darüber hinaus umfassend:
a. eine Probe des Organismus aus seiner natürlichen Umgebung zu entnehmen;
b. mitochondriales genomisches Material aus dem entnommenen Organismus zu isolieren;
c. das Nukleotid an der polymorphen Stelle des isolierten genomischen Materials zu identifizieren;
d. die polymorphe Nukleotidstelle des isolierten mitochondrialen Genom-Materials mit einer standardmäßigen mitochondrialen Nukleinsäuresequenz von einem Mitglied der Spezies zu vergleichen, die bekannt dafür ist, Pyrethroid-empfindlich zu sein, wobei der entnommene Organismus als resistent gegen Pyrethroide bestimmt wird, wenn sich das Nukleotid an der polymorphen Stelle von dem Nukleotid an der polymorphen Stelle des Standards unterscheidet.

8. Verfahren nach Anspruch 7, wobei der Schritt c) eine durch Polymerasekettenreaktion erfolgenden Nukleinsäureamplifikation umfasst.

9. Verfahren nach Anspruch 7, wobei der Schritt c) umfasst, die mitochondriale DNA-Sequenz des entnommenen Organismus mit einem Detektionsreagens in Kontakt zu bringen und zu bestimmen, welches Nukleotid an der polymorphen Stelle vorhanden ist.

10. Verfahren nach Anspruch 7, wobei der Schritt c) anhand von SNP-spezifischer Sondenhybridisierung, SNP-spezifischer Primer Extension, SNP-spezifischer Amplifikation, Sequenzierung, 5'-Nukleaseverdauung, Molecular Beacon Assay, Oligonukleotid-Ligations-Assay, Größenanalyse, Einzelstrang-Konformationspolymorphismus-Analyse oder denaturierende Gradientengelelektrophorese durchgeführt wird.

11. Verfahren zum Identifizieren eines Einzelnukleotidpolymorphismus, der auf Pyrethroid-Resistenz bei Seeläusen hinweist, die folgenden Schritte umfassend:
i. Bestimmen, ob die Spezies, zu der die Seeläuse gehören, Fälle von Pyrethroid-Resistenz aufweist,
ii. Bestimmen, ob die Pyrethroid-Resistenz innerhalb der Spezies von den weiblichen Mitgliedern an ihre Nachkommen vererbt wird,
iii. im Falle, dass die Pyrethroid-Resistenz innerhalb der Spezies von den weiblichen Mitgliedern an ihre Nachkommen vererbt wird, mitochondriale DNA von einem Mitglied der Spezies zu isolieren, das bekannt dafür ist, resistent gegen Pyrethroide zu sein, und die isolierte DNA mit der mitochondrialen DNA von Mitgliedern der Spezies zu vergleichen, die dafür bekannt ist, Pyrethroid-empfindlich zu sein,
iv. einen oder mehrere Einzelnukleotidpolymorphismen in der mitochondrialen DNA des resistenten Mitglieds der Spezies zu identifizieren,
v. eine Kontrolle durchzuführen, um zu bestimmen, ob die Einzelnukleotidpolymorphismen auf Pyrethroid-Resistenz hinweisen.

12. Verfahren nach Anspruch 11, wobei der Einzelnukleotidpolymorphismus (SNP) in einer kodierenden Region eines Gens, das Cytochrom B kodiert, oder in einer kodierenden Region eines Gens vorhanden ist, das die Cytochromoxidase-Untereinheit I (COI) kodiert.

13. Verfahren nach Anspruch 11, wobei der Einzelnukleotidpolymorphismus (SNP) in einem regulatorischen, nicht kodierenden Teil der mitochondrialen DNA vorhanden ist.

14. Verfahren nach einem der Ansprüche 11 - 13, wobei es sich bei den Seeläusen um L. salmonis, Caligus elongatus oder Caligus rogercresseyi handelt.

15. Verfahren nach einem der Ansprüche 11 - 14, wobei es sich bei dem Schädling um ein Mitglied einer Spezies mit einer Geschlechtsbestimmung des Typs ZZ-ZO handelt.

## Revendications

1. Procédé *in vitro* de détection d'une résistance aux pyréthrinoïdes chez un pou de mer, comprenant les étapes consistant à déterminer que le pou de mer est un membre d'une espèce qui exhibe une hérédité de résistance aux pyréthrinoïdes transmise par les membres femelles de l'espèce, à identifier un site polymorphe correspondant à un polymorphisme d'un seul nucléotide (SNP) associé à une résistance aux pyréthrinoïdes dans l'ADN mitochondrial de l'espèce, et à détecter le nucléotide au niveau du site polymorphe dans l'organisme devant être analysé.

2. Procédé selon la revendication 1, dans lequel le polymorphisme d'un seul nucléotide (SNP) est présent dans une région codante d'un gène codant le cytochrome B ou dans une région codante d'un gène codant la sous-unité I de la cytochrome oxydase (COI).

3. Procédé selon la revendication 1, dans lequel le polymorphisme d'un seul nucléotide (SNP) est présent dans une partie non codante régulatrice de l'ADN mitochondrial.

4. Procédé selon l'une quelconque des revendications précédentes, dans lequel le pou de mer est L. salmonis, Caligus elongatus ou Caligus rogercresseyi.

5. Procédé selon l'une quelconque des revendications précédentes, dans lequel le pou de mer est un membre d'une espèce présentant une détermination sexuelle de type ZZ-ZO.

6. Procédé selon l'une quelconque des revendications précédentes, dans lequel le pou de mer est un Siphonostomatoida.

7. Procédé selon l'une quelconque des revendications précédentes, comprenant en outre
a. la collecte d'un échantillon de l'organisme à partir de son environnement naturel ;
b. l'isolement du matériel génomique mitochondrial à partir de l'organisme collecté ;
c. l'identification du nucléotide au niveau du site polymorphe du matériel génomique isolé ;
d. la comparaison du site de polymorphisme nucléotidique du matériel génomique mitochondrial isolé avec une séquence d'acide nucléique mitochondriale standard issue d'un membre de l'espèce connu pour être sensible aux pyréthrinoïdes, où il est déterminé que l'organisme collecté est résistant aux pyréthrinoïdes si le nucléotide au niveau du site polymorphe est différent du nucléotide au niveau du site polymorphe du standard.

8. Procédé selon la revendication 7, dans lequel l'étape c) comprend une amplification d'acide nucléique réalisée par une réaction en chaîne par polymérase.

9. Procédé selon la revendication 7, dans lequel l'étape c) comprend la mise en contact de la séquence d'ADN mitochondrial de l'organisme collecté avec un réactif de détection, et la détermination du nucléotide qui est présent au niveau du site polymorphe.

10. Procédé selon la revendication 7, dans lequel l'étape c) est réalisée en utilisant une hybridation de sonde spécifique d'un SNP, une extension d'amorce spécifique d'un SNP, une amplification spécifique d'un SNP, un séquençage, une digestion par une nucléase 5', un essai avec phase moléculaire, un essai de ligature d'oligonucléotide, une analyse de taille, une analyse de polymorphisme de conformation simple brin, ou une électrophorèse sur gel en gradient dénaturant.

11. Procédé d'identification d'un polymorphisme d'un seul nucléotide indiquant une résistance aux pyréthrinoïdes chez un pou de mer, comprenant les étapes consistant à :
i. déterminer si l'espèce à laquelle appartient le pou de mer exhibe des incidents de résistance aux pyréthrinoïdes,
ii. déterminer si ladite résistance aux pyréthrinoïdes est héréditaire au sein de l'espèce en étant transmise par les membres femelles à leur descendance,
iii. dans la situation où ladite résistance aux pyréthrinoïdes est héréditaire au sein de l'espèce en étant transmise par les membres femelles à leur descendance, alors isoler l'ADN mitochondrial d'un membre de l'espèce connu pour être résistant aux pyréthrinoïdes et comparer ledit ADN isolé avec l'ADN mitochondrial des membres de l'espèce connus pour être sensibles aux pyréthrinoïdes,
iv. identifier un ou plusieurs polymorphismes d'un seul nucléotide dans l'ADN mitochondrial du membre résistant de l'espèce,
v. effectuer un contrôle afin de déterminer si lesdits polymorphismes d'un seul nucléotide sont indicatifs d'une résistance aux pyréthrinoïdes.

12. Procédé selon la revendication 11, dans lequel le polymorphisme d'un seul nucléotide (SNP) est présent dans une région codante d'un gène codant le cytochrome B ou dans une région codante d'un gène codant la sous-unité I de la cytochrome oxydase (COI).

13. Procédé selon la revendication 11, dans lequel le polymorphisme d'un seul nucléotide (SNP) est présent dans une partie non codante régulatrice de l'ADN mitochondrial.

14. Procédé selon l'une quelconque des revendications 11 à 13, dans lequel le pou de mer est L. salmonis, Caligus elongatus ou Caligus rogercresseyi.

15. Procédé selon l'une quelconque des revendications 11 à 14, dans lequel le nuisible est un membre d'une espèce présentant une détermination sexuelle de type ZZ-ZO.
